# EUROPÄISCHE PATENTSCHRIFT

(11) **EP 0 665 825 B1**
(45) Veröffentlichungstag und Bekanntmachung des Hinweises auf die Patenterteilung: **15.01.1997**
(21) Anmeldenummer: 93922520.7
(22) Anmeldetag: 06.10.1993
(51) Int. Cl.: C07B 37/04, C09K 19/34, C07D 239/26, C07D 239/80, C07D 213/24, C07D 213/61, C07D 401/04, C07D 401/14

(54) **VERFAHREN ZUR KREUZKUPPLUNG VON AROMATISCHEN BORONSÄUREN MIT AROMATISCHEN HALOGENVERBINDUNGEN ODER PERFLUORALKYLSULFONATEN**
METHOD OF CROSSLINKING AROMATIC BORON ACIDS WITH AROMATIC HALOGEN COMPOUNDS OR PERFLUOROALKYL SULPHONATES
PROCEDE DE RETICULATION D'ACIDES BORIQUES AROMATIQUES AVEC DES COMPOSES HALOGENES AROMATIQUES OU DES SULFONATES D'ALKYLE PERFLUORIQUE

(30) Priorität: 26.10.1992 DE 4236103
(43) Veröffentlichungstag der Anmeldung: 09.08.1995
(73) Patentinhaber: HOECHST AKTIENGESELLSCHAFT, 65929 Frankfurt am Main (DE)
(72) Erfinder: SCHLOSSER, Hubert, D-61479 Glashütten (DE); WINGEN, Rainer, D-65795 Hattersheim am Main (DE); MANERO, Javier, D-65931 Frankfurt am Main 80 (DE)
(86) Internationale Anmeldenummer: EP9302733
(87) Internationale Veröffentlichungsnummer: WO9410105

(56) Entgegenhaltungen:
- WO-A-89/12039
- DE-A- 3 930 663
- TETRAHEDRON, (INCL. TETRAHEDRON REPORTS) Bd. 48, Nr. 37 , 11. September 1992 , OXFORD GB Seiten 8117 - 8126 NAJI M. ALI 'Palladium-catalysed cross-coupling reactions of arylboronic acids with pi-deficient heteroaryl chlorides'

## Beschreibung

Die Erfindung betrifft ein Verfahren zur Herstellung mehrkerniger aromatischer Verbindungen durch Kreuzkupplung von aromatischen Boronsäuren mit aromatischen Halogenverbindungen oder Perfluoralkylsulfonaten unter Katalyse durch metallisches, gegebenenfalls auf Trägermetall aufgetragenes Palladium.

Die palladiumkatalysierte Kreuzkupplungsreaktion von terminalen Alkinen und metallorganischen Alkyl-, Alkenyl-, Allyl- oder Arylverbindungen mit Alkyl-, Alkenyl-, Allyl- oder Arylhalogeniden bzw. - Sulfonaten wird seit einigen Jahren in steigendem Umfang in vielen Bereichen der organischen Synthese genutzt (siehe z.B. B.M. Trost, T.R. Verhoeven in: Comprehensive Organometallic Chemistry Volume 8, S. 799 ff., Pergamon Press, Oxford 1982).

Die Kreuzkupplung von metallierten Arylen mit aromatischen Halogeniden ist beispielsweise mit Grignard- und lithiumorganischen (siehe z.B. J.-F. Fauvarque and A. Jutard, Bull. Chim. Soc. Fr., 1976, 765, A. Sekiya and N. Ishikawa, J. Organomet. Chem., 1976, 118, 349, A. Sekija and N. Ishikawa, J. Organoment. Chem., 1977, 125, 281, M. Yamamura, I. Moritani and S.I. Murahashi, J. Organomet. Chem., 1975, 91, C39, S.I. Murahashi, M. Yamamura, K. Yanagiswa, N. Mita and K. Kondo, J. Org. Chem., 1979, 44, 2408, A. Minato, K. Tamao, T. Hayashi, K. Suzuki and M. Kumada, Tetrahedron Lett., 1980, 845.), zinkorganischen - (z.B. E. Negishi et al. J. Org. Chem. 42 (1977) 1822.) und zinnorganischen Reagenzien (z.B. M. Kosogi et al., Chem. Lett. 1977, 301.) durchgeführt worden.

Auch aromatische Borverbindungen, wie Boronsäuren und deren Derivate oder Borane, wurden bereits zur Kupplung mit aromatischen Halogenverbindungen bzw.- Perfluoralkylsulfonaten eingesetzt (siehe z. B. N. Miyaura, T. Yanagi, A. Suzuki in Synthetic Communications 11 (1981), S. 513 ff.; M.J. Sharp, W. Cheng, V. Snieckus in Tetrahedron Letters 28 (1987), S. 5093 ff.; G.W. Gray in J. Chem. Soc. Perkin Trans II, 1989, S. 2041 ff. und Mol. Cryst. Sig. Cryst. 172 (1989), S. 165 ff., 204 (1991), S. 43 ff. und S. 91 ff.; EP 0 449 015; WO 89/12039; WO 89/03821; EP 0 354 434).

Bei all diesen Verfahren handelt es sich um homogen katalysierte Prozesse unter Verwendung von Pd(0)-Komplexen, insbesondere Tetrakis-(triphenylphosphin)-palladium(0).
Der Nachteil dieser Verfahren liegt jedoch eindeutig bei den hohen Katalysatorkosten, die eine wirtschaftliche Überführung der Prozesse in einen größeren Produktionsmaßstab (kg, t) schwierig machen. Die homogene Reaktionsführung erschwert des weiteren eine effiziente Regenerierung des Palladium-Katalysators und kann leicht zu einer Kontamination des bei der Reaktion anfallenden Abfalls mit Palladium führen.

Es sind deshalb Verfahren entwickelt worden, die diese Probleme durch heterogenen Einsatz des Katalysators umgehen. In der EP-A- 0 152 450 ist die Kupplung von Grignard Reagenzien unter heterogener Pd(0)-Katalyse beschrieben. Wegen der hohen Reaktivität der magnesiumorganischen Komponente ist jedoch die Wahl der Ausgangsstoffe für dieses Verfahren stark eingeschränkt. In der deutschen Patentschrift 3 930 663 ist ein Verfahren zur Herstellung flüssigkristalliner Verbindungen beschrieben, bei dem Halogenide und metallorganische Verbindungen, darunter auch Boronsäuren, in inerten Lösungsmitteln unter Verwendung von metallischem, ggf. auf ein Trägermaterial aufgezogenem Palladium als Katalysator, ggf. in Gegenwart eines Metallalkoholats umgesetzt werden. Durch diese Vorgehensweise können zwar die Katalysatorkosten wesentlich gesenkt und das Palladium nach Beendigung der Umsetzungen leicht regeneriert werden, jedoch liefert dieses Verfahren das angestrebte Kupplungsprodukt vielfach nur in unbefriedigenden Ausbeuten.

Aufgabe der vorliegenden Erfindung war es somit, ein Verfahren zur Kupplung von aromatischen Boronsäuren mit aromatischen Halogenverbindungen oder Perfluoralkylsulfonaten zu finden, das die beschriebenen Nachteile der bisherigen Verfahren nicht aufweist.

Überraschenderweise wurde gefunden, daß bei der Umsetzung von aromatischen Boronsäuren mit aromatischen Halogenverbindungen oder Perfluoralkylsulfonaten in Gegenwart einer Base und katalytischer Mengen von metallischem, ggf. auf ein Trägermaterial aufgetragenem Palladium durch Zugabe katalytischer Mengen eines Liganden mehrkernige aromatische Verbindungen in hervorragenden Ausbeuten erhalten werden können.

Gegenstand der Erfindung ist somit ein Verfahren zur Herstellung von mehrkernigen aromatischen Verbindungen durch Kreuzkupplung von aromatischen Boronsäuren mit aromatischen Halogenverbindungen oder Perfluoralkylsulfonaten in Gegenwart von metallischem, gegebenenfalls auf ein Trägermaterial aufgebrachtem Palladium als Katalysator, dadurch gekennzeichnet, daß die Kupplung in Gegenwart eines Liganden aus der Gruppe Phosphine, Diketone und tertiäre Amine und einer Base durchgeführt wird.

Die Kupplungsreaktion verläuft chemoselektiv, so daß selbst elektrophile Gruppen wie Ester oder Nitrile den Verlauf der Reaktion nicht beeinträchtigen.

Das bei diesem Verfahren eingesetzte Katalysatorsystem, bestehend aus metallischem, ggf. auf ein Trägermaterial aufgetragenem Palladium und einem Liganden, greift auf im Handel kostengünstig erhältliche Komponenten zurück, die eine wirtschaftliche Prozeßführung erlauben. Darüber hinaus läßt sich das nach Beendigung der Reaktion als Feststoff anfallende Palladiummetall leicht abtrennen, regenerieren und wieder dem Katalysatorprozeß zuführen, wodurch eine zusätzliche Senkung der Verfahrenskosten und eine Entlastung der Abfallprodukte von Palladium erreicht wird.

Durch die erfindungsgemäße Zugabe von katalytischen Mengen eines Liganden läßt sich die Ausbeute an mehrkernigen aromatischen Verbindungen gegenüber Verfahren ohne Verwendung eines Liganden deutlich steigern und somit ein Ausbeuteoptimum für die Pd(O)-katalysierte Kreuzkopplung aromatischer Boronsäuren mit aromatischen Halogenverbindungen erreichen.

Zur Durchführung des erfindungsgemäßen Verfahrens werden die aromatische Boronsäure, die aromatische Halogenverbindung bzw. das Perfluoralkylsulfonat, die Base, die katalytische Menge metallischen, ggf. auf Trägermaterial aufgetragenen Palladiums und die katalytische Menge eines Liganden vorzugsweise in ein inertes Lösungsmittel oder inertes Lösungsmittelgemisch gegeben und bei einer Temperatur von 0° C bis 200° C, bevorzugt bei 30° C bis 170° C, besonders bevorzugt bei 50° C bis 150° C, insbesonders bevorzugt bei 60 bis 120° C, für einen Zeitraum von 1 h bis 100 h, bevorzugt 5 h bis 70 h, besonders bevorzugt 10 h bis 50 h, insbesonders bevorzugt 15 h bis 30 h, gerührt. Nach beendeter Umsetzung wird der als Feststoff anfallende Pd-Katalysator durch Filtration abgetrennt, das Rohprodukt vom Lösungsmittel bzw. den Lösungsmitteln befreit und anschließend nach dem Fachmann bekannten und dem jeweiligen Produkt angemessenen Methoden, z. B. durch Umkristallisation, Destillation, Sublimation, Zonenschmelzen, Schmelzkristallisation oder Chromatographie, gereinigt.

Für das erfindungsgemäße Verfahren geeignete Lösungsmittel sind beispielsweise Ether, z. B. Diethylether, Dimethoxymethan, Diethylenglykoldimethylether, Tetrahydrofuran, Dioxan, Diisopropylether, tert.-Butylmethylether, Kohlenwasserstoffe, z. B. Hexan, iso-Hexan, Heptan, Cyclohexan, Benzol, Toluol, Xylol, Alkohole, z. B. Methanol, Ethanol, 1-Propanol, 2-Propanol, Ethylenglykol, 1-Butanol, 2-Butanol, tert.-Butanol, Ketone, z. B. Aceton, Ethylmethylketon, iso-Butylmethylketon, Amide, z. B. Dimethylformamid, Dimethylacetamid, N-Methylpyrrolidon, Nitrile, z. B. Acetonitril, Propionitril, Butyronitril, Wasser und Mischungen derselben.

Bevorzugte Lösungsmittel sind Ether, wie Dimethoxyethan, Diethylenglykoldimethylether, Tetrahydrofuran, Dioxan, Diisopropylether, Kohlenwasserstoffe, wie Hexan, Heptan, Cyclohexan, Benzol, Toluol, Xylol, Alkohole, wie Methanol, Ethanol, 1-Propanol, 2-Propanol, 1-Butanol, 2-Butanol, Ethylenglykol, Ketone, wie Ethylmethylketon, iso-Butylmethylketon, Amide, wie Dimethylformamid, Dimethylacetamid, N-Methylpyrrolidon, Hexamethylphosphorsäuretriamid, Wasser und Mischungen derselben.

Besonders bevorzugte Lösungsmittel sind Ether, z. B. Dimethoxyethan, Tetrahydrofuran, Kohlenwasserstoffe, z. B. Cyclohexan, Benzol, Toluol, Xylol, Alkohole, z. B. Ethanol, 1-Propanol, 2-Propanol, Wasser und Mischungen derselben.

Insbesonders bevorzugt sind Dimethoxyethan, Benzol, Toluol, Ethanol, Wasser und Mischungen derselben.

Basen, die bei dem erfindungsgemäßen Verfahren vorzugsweise Verwendung finden sind Alkali- und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkoholate, sowie primäre, sekundäre und tertiäre Amine.

Besonders bevorzugt sind Alkali- und Erdalkalimetallhydroxide, Alkali- und Erdalkalimetallcarbonate und Alkalimetallhydrogencatonate. Insbesondere bevorzugt sind Alkalimetallhydroxide, Alkalimetallcarbonate und Alkalimetallhydrogencarbonate, wie Lithiumcarbonat, Natriumcarbonat, Kaliumcarbonat.
Die Base wird bei dem erfindungsgemäßen Verfahren bevorzugt mit einem Anteil von 100 bis 1000 Mol %, besonders bevorzugt 100 bis 500 Mol %, ganz besonders bevorzugt 150 bis 400 Mol%, insbesondere 180 bis 250 Mol %, bezogen auf die aromatische Boronsäure, eingesetzt.

Als Katalysator dient metallisches Palladium, vorzugsweise Palladium in pulverisierter Form oder auf einem Trägermaterial, z.B. Palladium auf Aktivkohle, Palladium auf Aluminiumoxid, Palladium auf Bariumcarbonat, Palladium auf Bariumsulfat, Palladium auf Aluminiumsilikaten, wie Montmorillonit, Palladium auf SiO₂ und Palladium auf Calciumcarbonat, jeweils mit einem Palladiumgehalt von 0,5 bis 10 Gew.-%, besonders bevorzugt Palladium in pulverisierter Form, sowie Palladium auf Aktivkohle, Palladium auf Barium- und Calciumcarbonat, und Palladium auf Bariumsulfat, jeweils mit einem Palladiumgehalt von 0,5 bis 10 Gew.-%, insbesondere Palladium auf Aktivkohle mit einem Palladiumgehalt von 10 Gew.-%.
Es können auch Katalysatoren eingesetzt werden, die neben Palladium und dem Trägermaterial weitere Dotierstoffe, z.B. Blei (Lindlar-Katalysator), enthalten.

Der metallische Palladiumkatalysator wird bei dem erfindungsgemäßen Verfahren mit einem Anteil von 0,1 bis 10 Mol %, bevorzugt 0,2 bis 5 Mol %, besonders bevorzugt 0,5 bis 3 Mol %, insbesonders bevorzugt 0,5 bis 1,5 Mol %, bezogen auf die aromatische Halogenverbindung oder das Perfluoralkylsulfonat, eingesetzt.

Für das erfindungsgemäße Verfahren geeignete Liganden sind beispielsweise Phosphine, wie Trialkylphosphine, Tricycloalkylphosphine, Triarylphosphine, wobei die drei Substituenten am Phosphor gleich oder verschieden, chiral oder achiral sein können und wobei einer oder mehrere der Liganden die Phosphorgruppen mehrerer Phosphine verknüpfen können und wobei ein Teil dieser Verknüpfung auch ein oder mehrere Metallatome sein können.
Beispiele für im Rahmen des erfindungsgemäßen Verfahrens verwendbare Phosphine sind Trimethylphosphin, Tributylphosphin, Tricyclohexylphosphin, Triphenylphosphin, Tritolylphosphin, Tris-(4-dimethylaminophenyl)-phosphin, Bis(diphenylplhosphino)methan, 1,2-Bis(diphenylphosphino)ethan, 1,3-Bis(diphenylphosphino)propan und 1,1'-Bis(diphenylphosphino)-ferrocen. Weitere geeignete Liganden sind beispielsweise Diketone, z. B. Acetylaceton und Octafluoracetylaceton und tert. Amine, z. B. Trimethylamin, Triethylamin, Tri-n-propylamin und Triisopropylamin.

Bevorzugte Liganden sind Phosphine und Diketone, besonders bevorzugt sind Phosphine.
Ganz besonders bevorzugte Liganden sind Triphenylphosphin, 1,2-Bis(diphenylphosphino)ethan, 1,3-Bis(diphenylphosphino)propan und 1,1'-Bis(diphenylphosphino)-ferrocen, insbesondere Triphenylphosphin.
Der Ligand wird bei dem erfindungsgemäßen Verfahren mit einem Anteil von 0,1 bis 20 Mol %, bevorzugt 0,2 bis 15 Mol %, besonders bevorzugt 0,5 bis 10 Mol %, insbesonders bevorzugt 1 bis 6 Mol %, bezogen auf die aromatische Halogenverbindung oder das Perfluoralkylsulfonat, eingesetzt.
Es können gegebenenfalls auch Mischungen zweier oder mehr verschiedener Liganden eingesetzt werden.

Eine Klasse von Ausgangsverbindungen für das erfindungsgemäße Verfahren sind aromatische Boronsäuren, vorzugsweise solche der Formel II,

R¹(-A¹)ₖ(-M¹)ₗ-A²-B(OH)₂ (II)

wobei R¹, A¹, A², M¹, k und l die folgende Bedeutung haben:
R¹ ist Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, 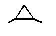 oder -Si(CH₃)₂- ersetzt sein können, und wobei auch ein oder mehrere H-Atome des Alkylrestes durch F, Cl, Br oder CN substituiert sein können.
A¹ ist 1,4-Phenylen, Pyrazin-2,5-diyl, Pyridazin-3,6-diyl, Pyridin-2,5-diyl, Pyrimidin-2,5-diyl, wobei eine oder zwei H-Atome durch Halogenatome, Cyano- und oder Methylgruppen substituiert sein können, trans-1,4-Cyclohexylen, worin eine oder zwei nicht benachbarte CH₂-Gruppen durch -O- oder -S- ersetzt sein können und worin ein oder zwei H-Atome durch Halogenatome, Cyano- und/oder Methylgruppen substituiert sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Naphthalin-2,6-diyl, Bicyclo[2.2.2]octan-1,4-diyl, 1,3-Dioxaborinan-2,5-diyl oder trans-Dekalin-2,6-diyl,
A² ist 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome, Cyano- und/oder Methylgruppen substituiert sein können, 1,3,4-Thiadiazol-2,5-diyl, 1,3-Thiazol-2,4-diyl, 1,3-Thiazol-2,5-diyl, Thiophen-2,4-diyl, Thiophen-2,5-diyl oder Naphthalin-2,6-diyl sein können,
M¹ ist
   -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-,-CH₂CH₂CH₂-O-, -OCH₂CH₂CH₂-, -CH₂CH₂CO-O-, -O-COCH₂CH₂-, und
k, l bedeuten jeweils unabhängig voneinander Null oder Eins.

Bevorzugt bedeutet R¹, Benzyloxy, H, F, Cl, -CF₃, OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -CO-, -CO-O-, -OCO- 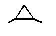 -O-CO-O-, -CH=CH-, -C≡C-,
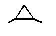 oder -Si(CH₃)₂- ersetzt sein können, und wobei auch ein oder mehrere H-Atome des Alkylrests durch F, Cl oder CN substituiert sein können.

Besonders bevorzugt bedeutet R¹ Benzyloxy, H oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -CH = CH-, -C≡C-,
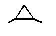 oder -Si(CH₃)₂- ersetzt sein können.

Bevorzugt bedeutet A¹ 1,4-Phenylen, Pyrazin-2,5-diyl, Pyridazin-3,6-diyl, Pyridin-2,5-diyl, Pyrimin-2,5-diyl, wobei ein oder zwei H-Atome durch Halogenatome substituiert sein können, trans-1,4-Cyclohexylen, worin eine oder zwei nicht benachbarte -CH₂-Gruppen durch -O- ersetzt sein können, (1,3,4)-Thiadiazol-2,5-diyl, Napthalin-2,6-diyl oder Bicyclo[2.2.2]octan-1,4-diyl.

Besonders bevorzugt bedeutet A¹ 1,4-Phenylen, 2-Fluor-1,4-Phenylen, 2,3-Difluor-1,4-Phenylen, 2,6-Difluor-1,4-Phenylen, 2,5-Difluor-1,4-Phenylen, sowie Pyridin-2,5-diyl, Pyrimidin-2,5-diyl, wobei ein oder zwei H-Atome durch Fluor substituiert sein können oder trans-1,4-Cyclohexylen.

Bevorzugt bedeutet A² 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome ersetzt sein können oder Naphthaiin-2,6-diyl.

Besonders bevorzugt bedeutet A² 1,4-Phenylen, 2-Fluor-1,4-Phenylen, 2,3-Difluor-1,4-phenylen, 2,6-Difluor-1,4-Phenylen, 2,5-Difluor-1,4-phenylen, sowie Pyridin-2,5-diyl, wobei ein oder zwei H-Atome durch F substituiert sein können oder Naphthalin-2,6-diyl.

Bevorzugt bedeutet M¹ -O-, -CO-, -CO-O-, -O-CO-, -O-CO-O-, -CH₂-O-, -O-CH₂-, -CH₂-CH₂-, -CH=CH-, -C≡C-, -CH₂CH₂CH₂-O-, -O-CH₂CH₂CH₂-, -CH₂CH₂CO-O- oder -O-CO-CH₂CH₂-.

Besonders bevorzugt bedeutet M¹ -O-, -CH₂-O-, -O-CH₂-, -CH₂CH₂-, -CH=CH- oder -C≡C-.

Insbesondere bevorzugt sind die nachfolgend aufgeführten aromatischen Boronsäuren der Formel IIa bis IIh: 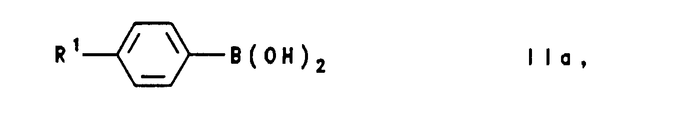 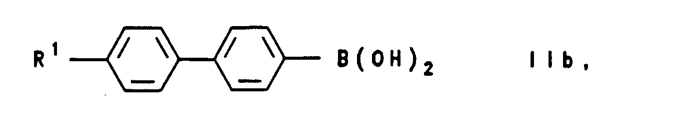 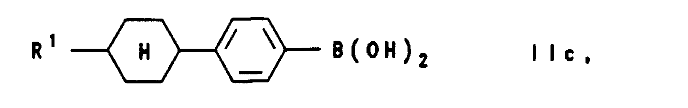 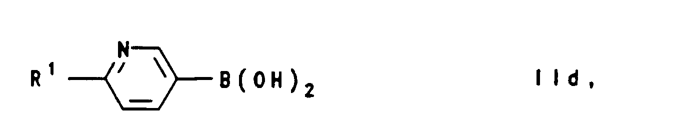 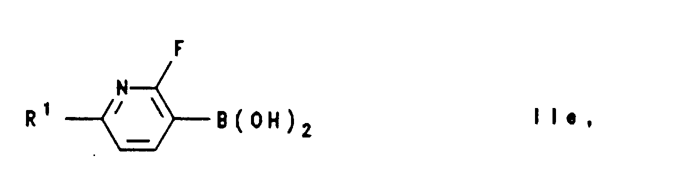 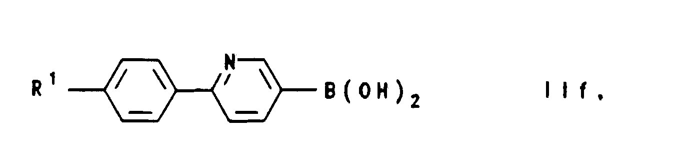 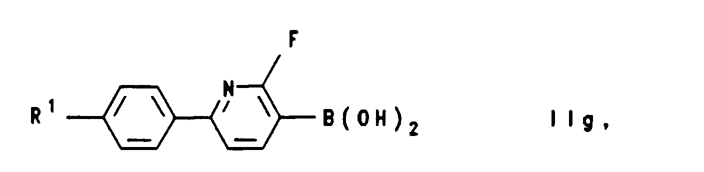 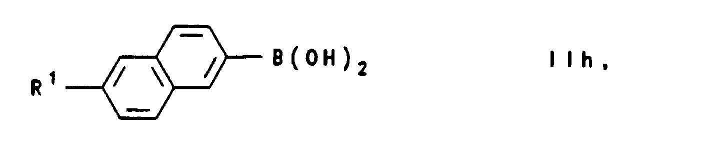 wobei R¹, Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy bedeuten.

Die verwendeten aromatischen Boronsäuren vorzugsweise solche der Formel II, sind entweder bekannt oder können nach bekannten Methoden, wie beispielsweise in Houben Weyl Methoden der Organischen Chemie, Georg Thieme-Verlag, Stuttgart, Band 13/3a beschrieben, hergestellt werden. So ist es beispielsweise möglich, aus aromatischen Alkalimetall- und Magnesiumverbindungen durch Umsetzung mit Trialkoxyboranen und anschließender Hydrolyse Boronsäuren, vorzugsweise solche der Formel II, zu erhalten.

Die zweite Klasse von Ausgangsverbindungen für das erfindungsgemäße Verfahren sind aromatische Halogenverbindungen oder aromatische Perfluoralkylsulfonate, vorzugsweise solche der Formel III,

X-A³(-M²)ₘ(-A⁴)ₙ-R² (III),

wobei R², A³, A⁴, M², X m und n die folgende Bedeutung haben.
R² ist Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-,
   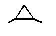 oder -Si(CH₃)₂- ersetzt sein können, und wobei auch ein oder mehrere H-Atome des Alkylrestes durch F, Cl, Br oder CN substituiert sein können,
A⁴ ist 1,4-Phenylen, Pyrazin-2,5-diyl, Pyridazin-3,6-diyl, Pyridin-2,5-diyl, Pyrimidin-2,5-diyl, wobei eine oder zwei H-Atome durch Halogenatome, Cyano- und oder Methylgruppen substituiert sein können, trans-1,4-Cyclohexylen, worin eine oder zwei nicht benachbarte CH₂-Gruppen durch -O- oder -S- ersetzt sein können und worin ein oder zwei H-Atome durch Halogenatome, Cyano- und/oder Methylgruppen substituiert sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Naphthalin-2,6-diyl, Bicyclo [2.2.2]octan-1,4-diyl, 1,3-Dioxaborinan-2,5-diyl oder trans-Dekalin-2,6-diyl,
A³ ist 1,4-Phenylen, Pyrazin-2,5-diyl, Pyridazin-3,6-diyl, Pyridin-2,5-diyl, Pyrimidin-2,5-diyl, wobei auch ein oder zwei H-Atome durch Halogenatome, Cyano- und/oder Methylgruppen substituiert sein können, 1,3,4-Thiadiazol-2,5-diyl, 1,3-Thiazol-2,4-diyl, 1,3-Thiazol-2,5-diyl, Thiophen-2,4-diyl, Thiophen-2,5-diyl oder Naphthalin-2,6-diyl,
M² ist
   -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-, -CH₂CH₂CH₂-O-, -OCH₂CH₂CH₂-, -CH₂CH₂CO-O-, -O-COCH₂CH₂-, und
m, n bedeuten jeweils unabhängig voneinander Null oder Eins.

Bevorzugt bedeutet R² Benzyloxy, H, F, Cl, Br, -CN, -CF₃, -OCF₃ oder ein geradkettiger oder verzweigter (mit oder ohne asymmetrisches C-Atom) Alkylrest mit 1 bis 18 C-Atomen, wobei auch eine oder zwei nicht benachbarte -CH₂- Gruppen durch -O-, -CO-, -CO-O-, -O-CO-, -O-CO-O-, -CH=CH-, -C≡C-,
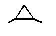 oder -Si(CH₃)₂- ersetzt sein können, und wobei auch ein oder mehrere H-Atome des Alkylrestes durch F, Cl oder CN substituiert sein können.

Besonders bevorzugt bedeutet R² Benzyloxy, H, Cl, Br oder ein geradkettiger oder verzweigter (mit oder ohne assymmetrisches C-Atom) Alkylrest mit 1 bis 18 C-Atomen, wobei auch eine oder zwei nicht benachtbarte -CH₂-Gruppen durch -O-, -CO-, -CO-O-, -O-, -O-CO-, -O-CO-O-, -CH =CH-, -C≡C-,
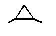 oder -Si(CH₃)-ersetzt sein können.

Bevorzugt bedeutet A³ 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome ersetzt sein können, 1,3,4-Thiadiazol-2,5-diyl oder Naphthalin-2,6-diyl.

Besonders bevorzugt bedeutet A³ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyrazin-2,5-diyl, Pyridazin-2,5-diyl, Pyrimidin-2,5-diyl, wobei ein oder zwei H-Atome durch Halogenatome ersetzt sein können oder Naphthalin-2,6-diyl.

Bevorzugt bedeutet A⁴ 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome substituiert sein können, trans-1,4-Cyclohexylen, worin eine oder zwei nicht benachbarte CH₂-Gruppen durch -O- ersetzt sein können, (1,3,4)-Thiadiazol-2,5-diyl, Napthalin-2,6-diyl oder Bicydo[2.2.2]octan-1,4-diyl.

Besonders bevorzugt bedeutet A⁴ 1,4-Phenylen, 2-Fluor-1,4-phenylen, 2,3-Difluor-1,4-phenylen, 2,6-Difluor-1,4-phenylen, 2,5-Difluor-1,4-phenylen, sowie Pyridin-2,5-diyl, Pyrimidin-2,5-diyl, wobei ein oder zwei H-Atome durch Fluor substituiert sein können oder trans-1,4-Cyclohexylen.

Bevorzugt bedeutet M² -O-, -CO-, -CO-O-, -O-CO-, -O-CO-O-, -CH₂-O-, -O-CH₂-, -CH₂CH₂-, -CH=CH-, -C≡C-, -CH₂CH₂CH₂-O-, -O-CH₂CH₂CH₂-, -CH₂CH₂CO-O-oder -O-CO-CH-₂CH₂-.

Besonders bevorzugt bedeutet M² -O-, -CO-, -CO-O-, -O-CO-, -O-CO-O-, -CH₂-O-,-O-CH₂-, -CH₂CH₂-, -CH=CH-, -C≡C-, -CH₂CH₂CH₂-O-, -O-CH₂CH₂CH₂-,-CH₂CH₂CO-O- oder -O-CO-CH₂CH₂.

Bevorzugt bedeutet X Chlor, Brom oder OSO₂-CₚF₂ₚ₊₁, worin p einen ganzzahligen Wert von 1 bis 10 darstellt. Besonders bevorzugt bedeutet X Chlor oder Brom.

Insbesondere bevorzugt sind die nachfolgend aufgeführten aromatischen Halogenverbindungen der Formel III 1 bis III 24, 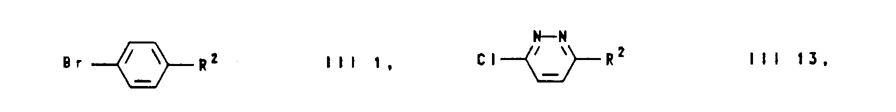 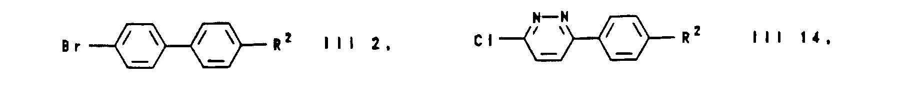 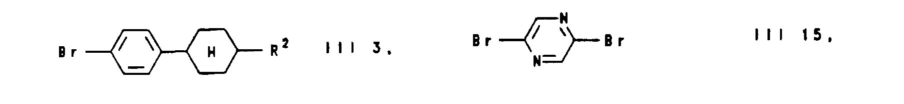 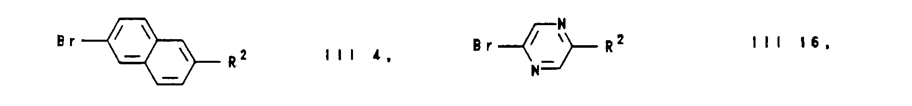 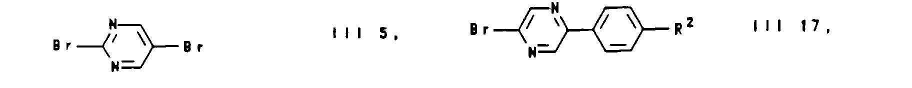 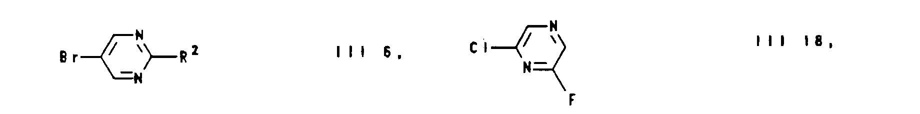 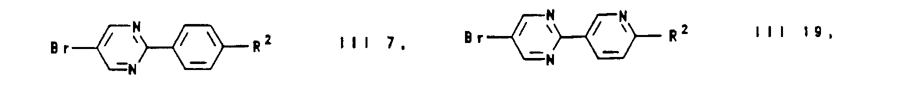 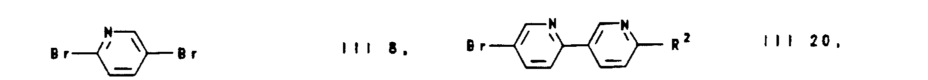 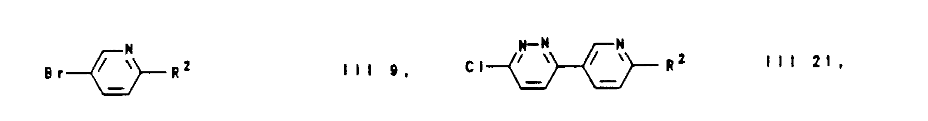 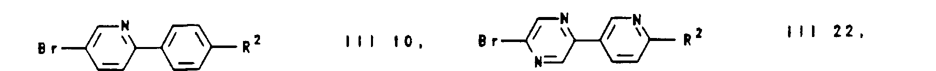 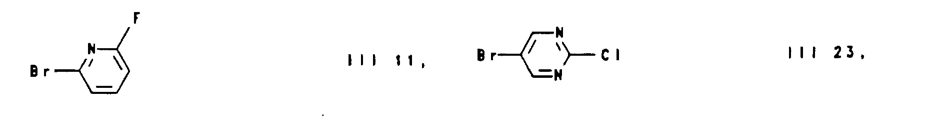 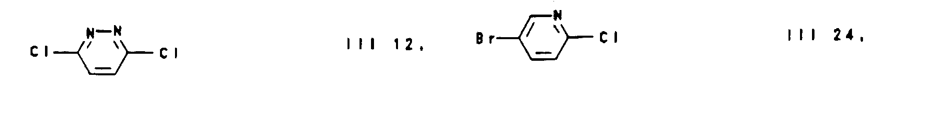 wobei R² Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy bedeuten.

Die aromatischen Halogenverbindungen und Perfluoralkylsulfonate, vorzugsweise die der allgemeinen Formel III, sind entweder bekannt oder können nach bekannten Methoden, wie beispielsweise in Houben Weyl, Methoden der Organischen Chemie, Georg Thieme Verlag, Stuttgart, Band 5/3 und 5/4 beschrieben, hergestellt werden. Beispielsweise lassen sich aromatische Halogenide dadurch erhalten, daß man in einem entsprechenden Diazoniumsalz die Diazoniumgruppe durch Chlor, Brom oder lod ersetzt.
Desweiteren lassen sich Hydroxy-Stickstoffheterocyclen mit Hilfe von Phosphortrihalogeniden und Phosphoroxytrihalogeniden in die entsprechenden Halogenide überführen.
Das erfindungsgemäße Verfahren zur Kreuzkupplung von aromatischen Boronsäuren mit aromatischen Halogenverbindungen oder Perfluoralkylsulfonaten kann ebenfalls zur Herstellung von Verbindungen der Formel III benutzt werden. Perfluoralkylsulfonate der Formel III, worin X OSO₂-CₙH₂ₙ₊₁ bedeutet, können durch Veresterung entsprechender Alkohole der Formel III, worin X eine Hydroxylgruppe bedeutet, mit Perfluoralkansulfonsäuren oder ihren reaktiven Derivaten hergestellt werden. Die entsprechenden Perfluoralkansulfonsäuren sind bekannt. Als reaktionsfähige Derivate der genannten Perfluoralkansulfonsäuren eignen sich insbesondere die Säurehalogenide, vor allem die Chloride und Bromide, ferner die Anhydride.

Produkte des erfindungsgemäßen Verfahrens sind mehrkemige aromatische Verbindungen.

Als bevorzugte Produkte des erfindungsgemäßen Verfahrens fallen Verbindungen der allgemeinen Formel I an,

R¹(-A¹)ₖ(-M¹)ₗ-A²-A³(-M²)ₘ(-A⁴)ₙ-R² (I)

wobei R¹ und R² unabhänigig voneinander Benzyloxy, H, F, Cl, Br, -NC, -CN, -CF₃, -OCF₃ oder ein geradkettiger oder verzweigter (mit oder ohne asymmetrisches C-Atom) Alkylrest mit 1 bis 18 C-Atomen sein können, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, 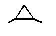 oder -Si(CH₃)₂- ersetzt sein können, und wobei auch ein oder mehrere H-Atome des Alkylrestes durch F, Cl, Br oder CN substituiert sein können,
A¹ und A⁴ jeweils unabhänigig voneinander 1,4-Phenylen, Pyrazin-2,5-diyl, Pyridazin-3,6-diyl, Pyridin-2,5-diyl, Pyrimidin-2,5-diyl, wobei eine oder zwei H-Atome durch Halogenatome, Cyano- und/oder Methylgruppen substituiert sein können, trans-1,4-Cyclohexylen, worin eine oder zwei nicht benachbarte CH₂-Gruppen durch -O- oder -S- ersetzt sein können und worin ein oder zwei H-Atome durch Halogenatome, Cyano- und/oder Methylgruppen substituiert sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Naphthalin-2,6-diyl, Bicyclo[2.2.2]octan-1,4-diyl, 1,3-Dioxaborinan-2,5-diyl oder trans-Dekalin-2,6-diyl,
A² und A³ jeweils unabhänig voneinander 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome, Cyano- und/oder Methylgruppen substituiert sein können, 1,3,4-Thiadiazol-2,5-diyl, 1,3-Thiazol-2,4-diyl, 1,3Thiazol-2,5-diyl, Thiophen-2,4-diyl, Thiophen-2,5-diyl oder Naphthalin-2,6-diyl sein können,
M¹ und M² jeweils unabhänig voneinander -O-, -S-, -CO-, -CO-O-, O-CO-, -CO-S, -S-CO-, -O-CO-O-, -CH₂-O-, -OCH₂-, -CH₂CH₂-, -CH=CH-, -C≡C-, -CH(CN)-CH₂-, -CH₂-CH(CN)-, -CH=N-, -N=CH-,-CH₂CH₂CH₂-O-, -OCH₂CH₂CH₂-, -CH₂CH₂CO-O-, -O-COCH₂CH₂- sein können,
und
k, l, m, n jeweils unabhängig voneinander Null oder Eins bedeuten.

Bevorzugte und besonders bevorzugte Varianten von R¹, R², A¹, A², A³, A⁴, M¹, M², k, l, m, n sind die in den Formeln II und III angegebenen.

Insbesondere bevorzugt sind die nachfolgend aufgeführten Verbindungen der Formel I 1 bis I 94 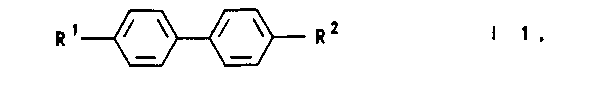 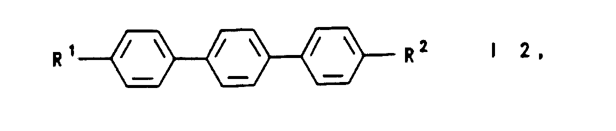 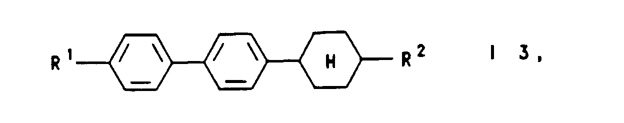 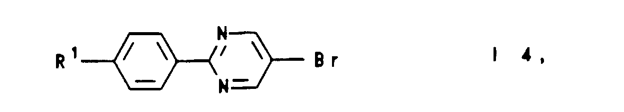 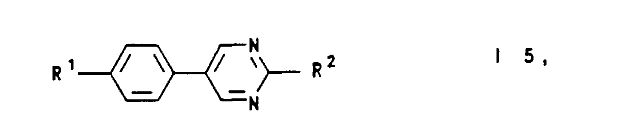 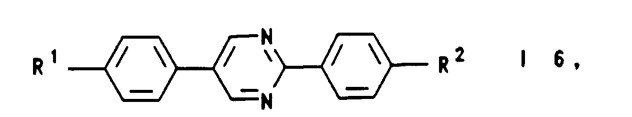 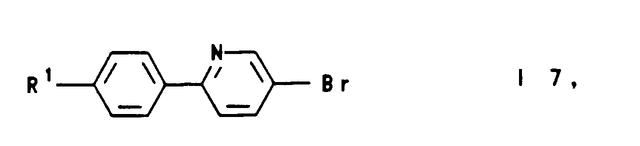 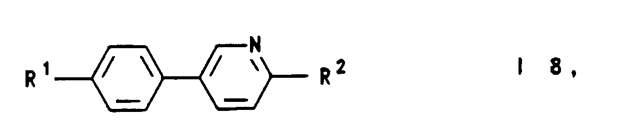 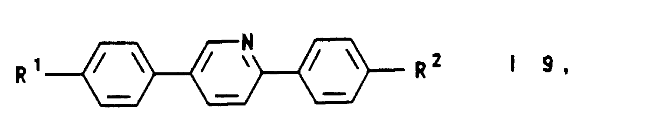 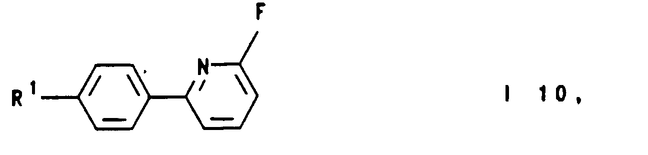 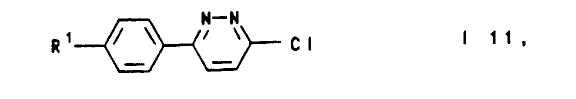 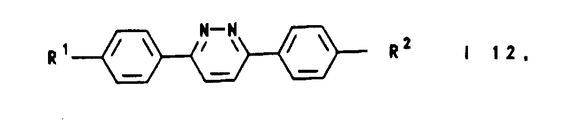 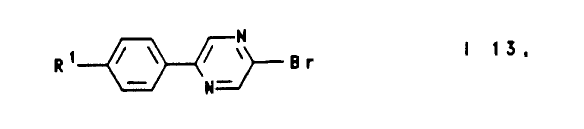 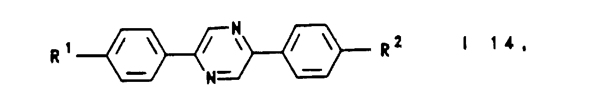 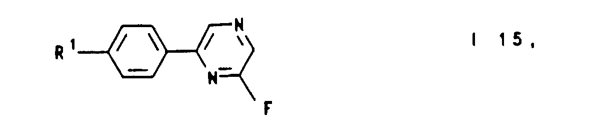 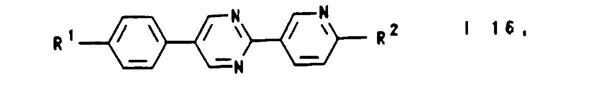 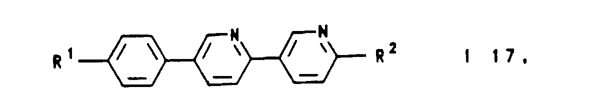 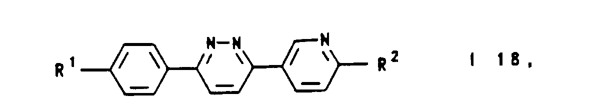 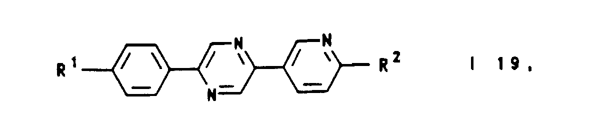 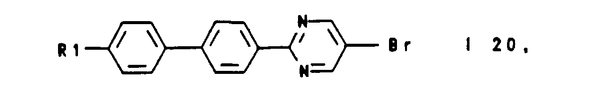 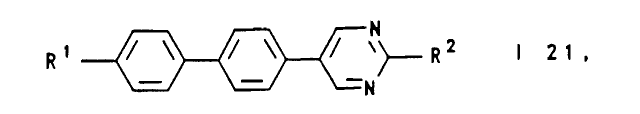 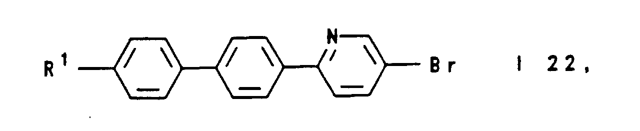 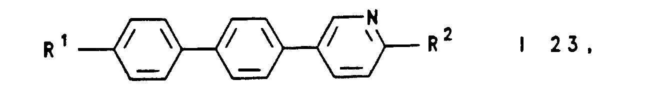 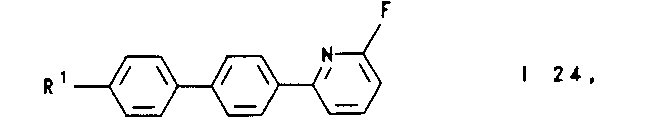 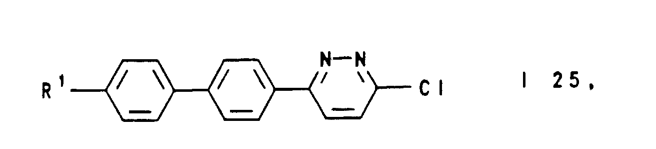 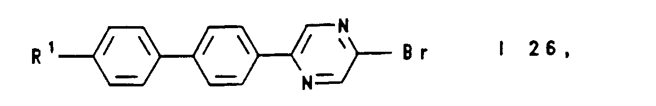 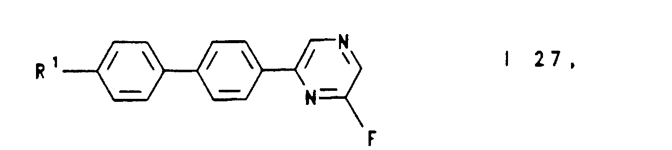 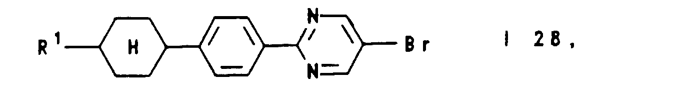 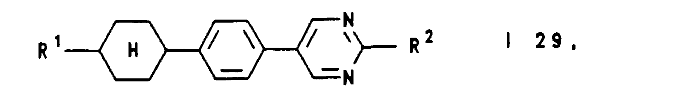 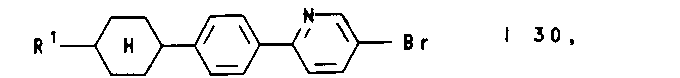 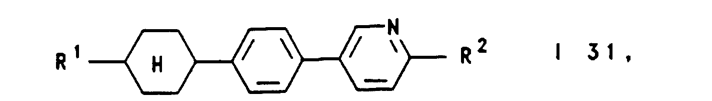 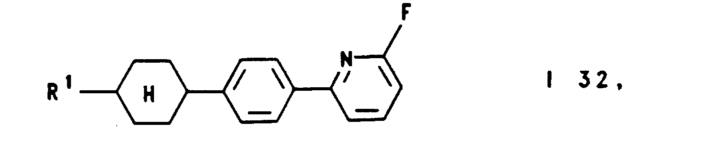 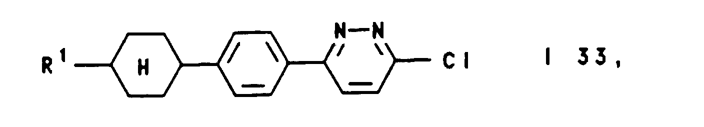 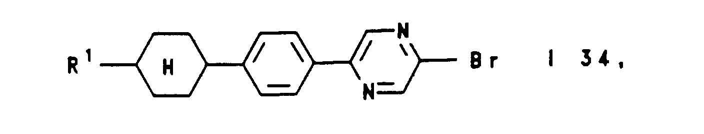 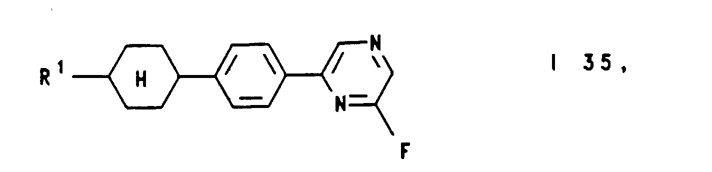 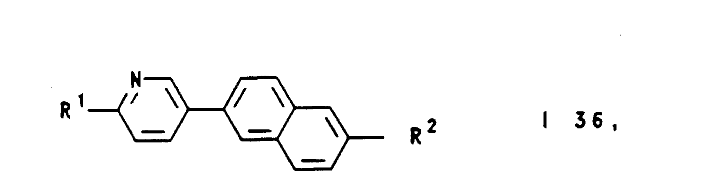 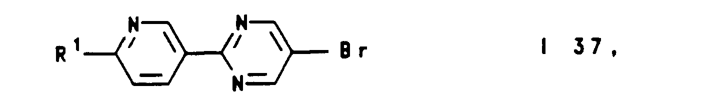 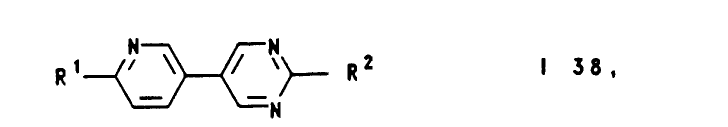 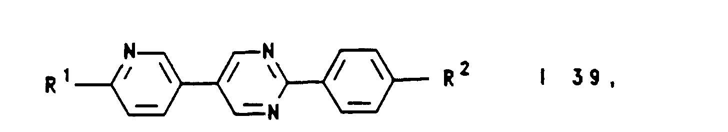 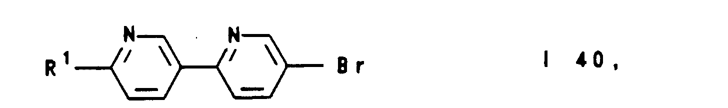 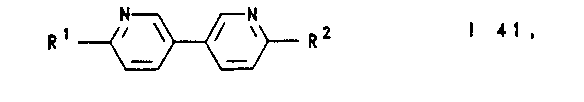 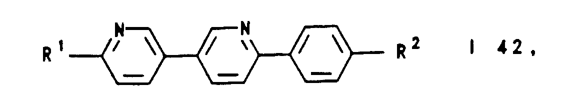 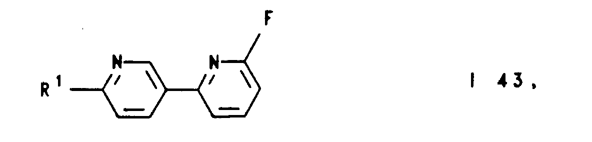 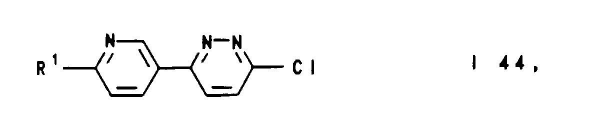 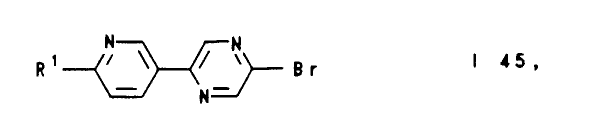 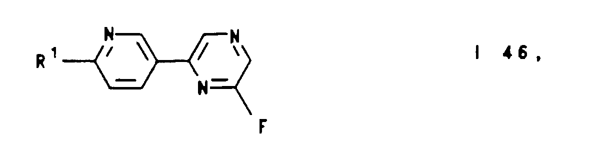 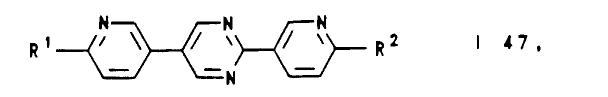 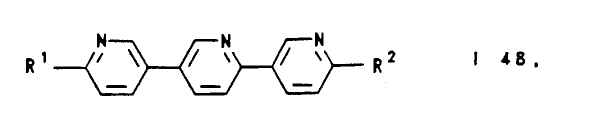 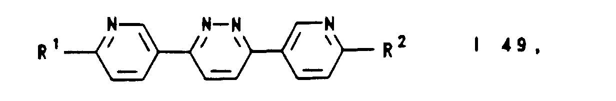 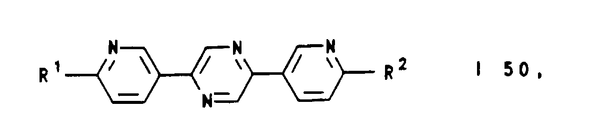 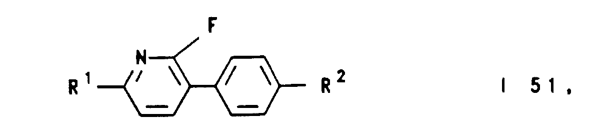 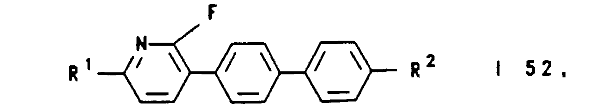 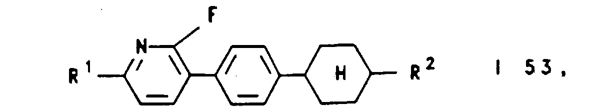 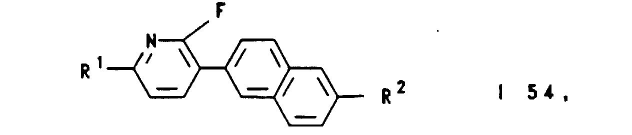 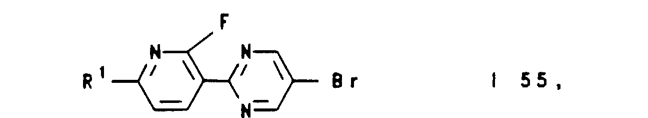 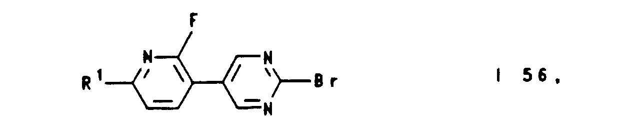 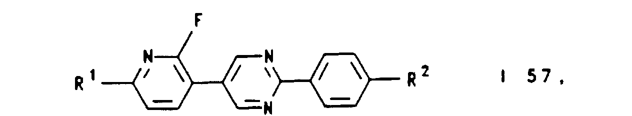 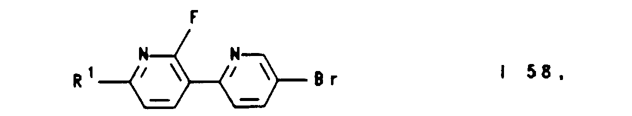 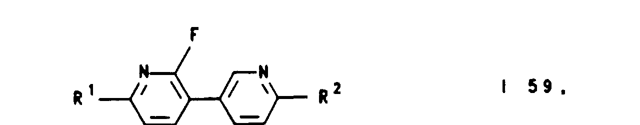 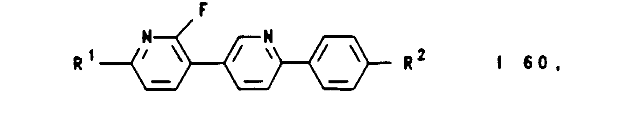 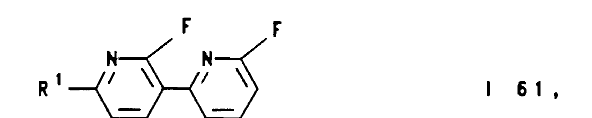 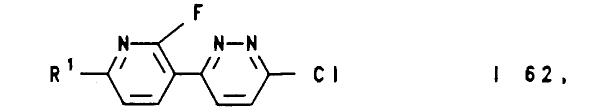 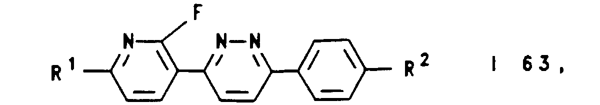 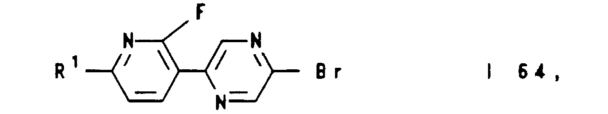 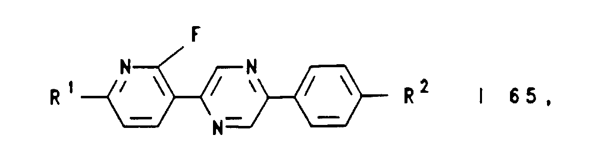 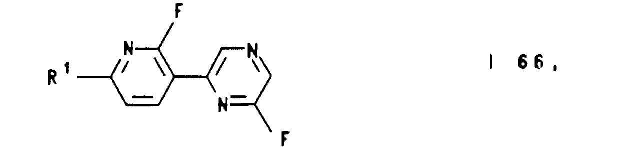 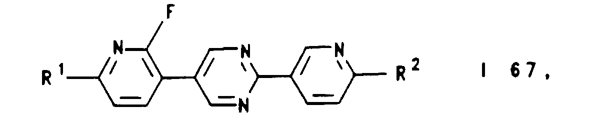 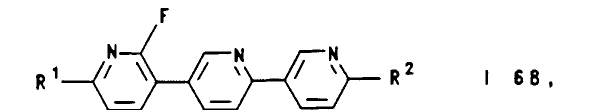 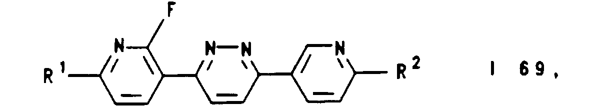 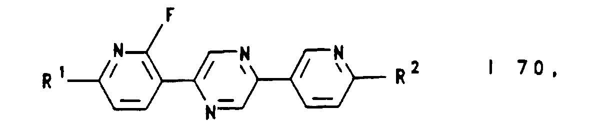 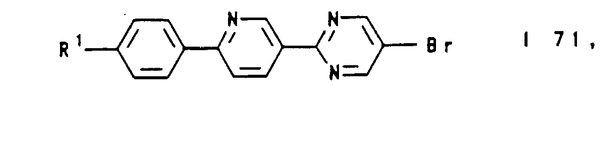 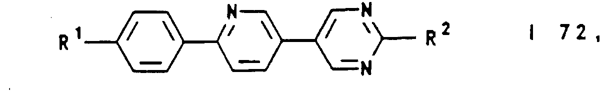 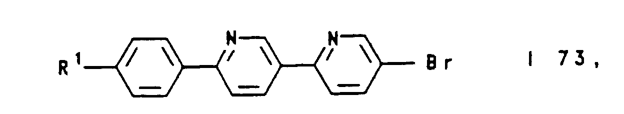 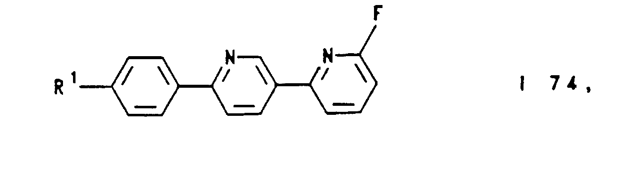 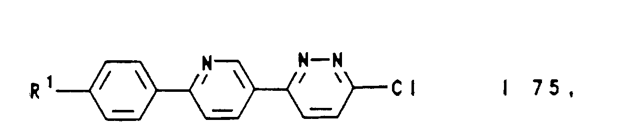 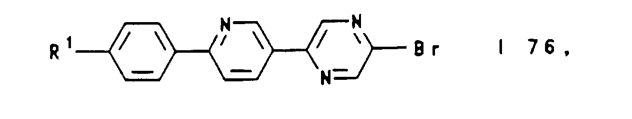 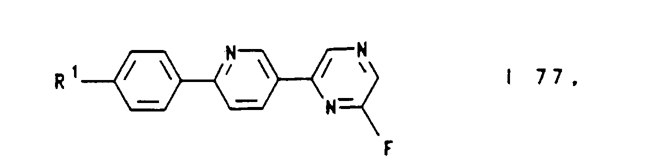 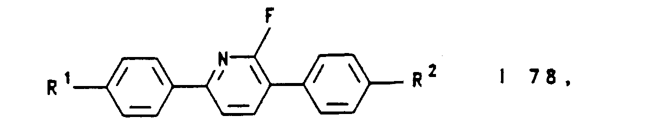 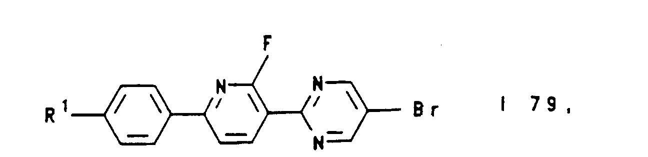 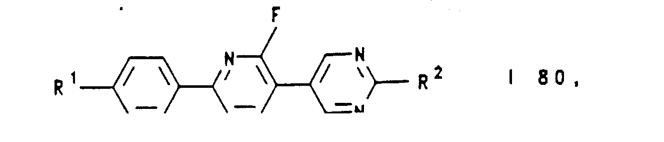 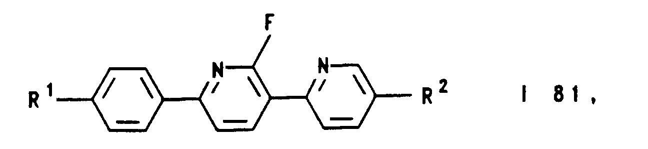 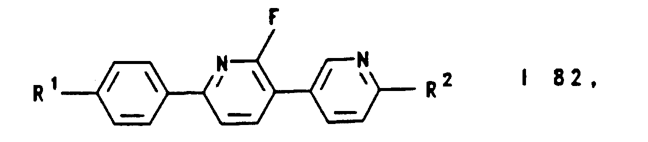 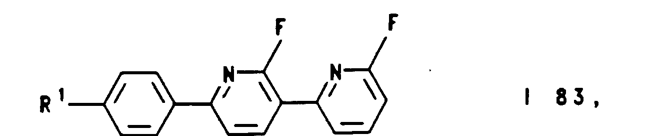 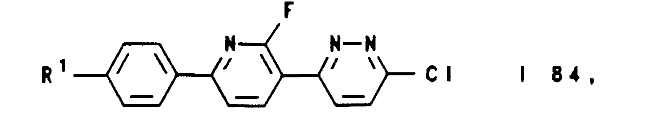 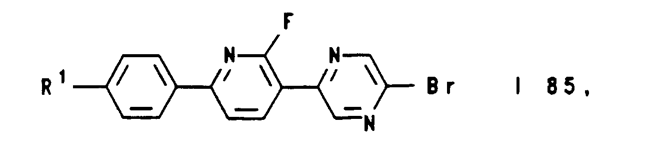 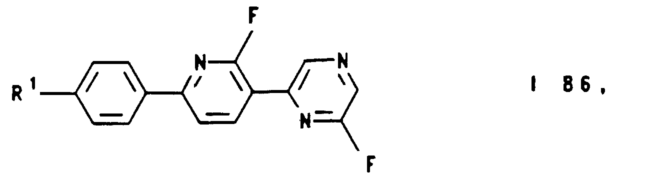 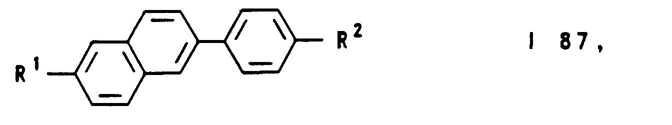 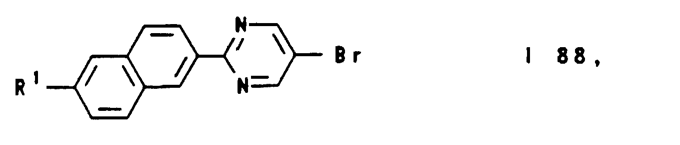 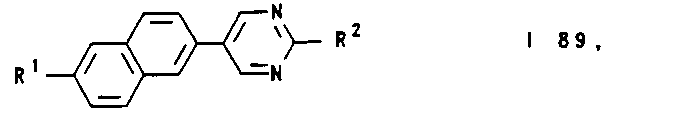 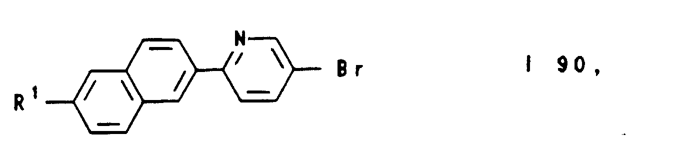 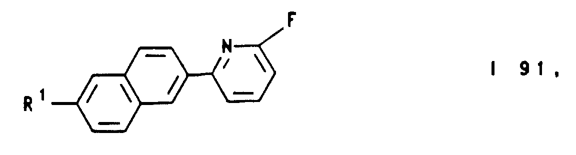 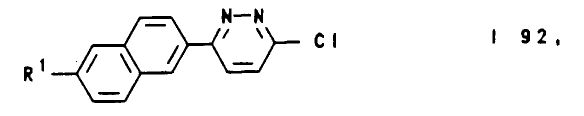 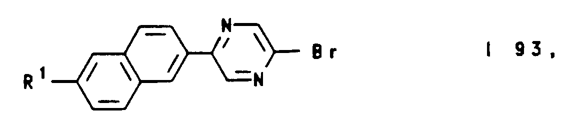 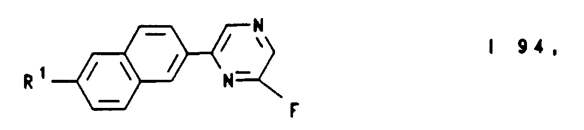 wobei R¹ und R² Benzyloxy, H, Methyl, Ethyl, Propyl, Butyl, Pentyl, Hexyl, Heptyl, Octyl, Nonyl, Decyl, Undecyl, Dodecyl, Tridecyl, Tetradecyl und Pentadecyl, sowie Methoxy, Ethoxy, Propoxy, Butoxy, Pentoxy, Hexoxy, Heptoxy, Octoxy, Nonoxy, Decoxy, Undecoxy, Dodecoxy, Tridecoxy, Tetradecoxy und Pentadecoxy bedeuten.

Die Verbindungen der Formel I eignen sich zum Einsatz als flüssigkristalline Materialien, oder können als Zwischenprodukte für die Herstellung weiterer flüssigkristalliner Verbindungen verwendet werden. Desweiteren kommen Verbindungen der Formel I als Vorprodukte für Pharmazeutika, Kosmetika, Fungizide, Herbizide, Insektizide, Farbstoffe, Detergenzien und Polymere, einschließlich als Zusatzstoffe derselben, in Betracht.

Die vorliegende Erfindung soll durch die nachfolgend beschriebenen Beispiele näher erläutert werden, ohne sie allerdings zu begrenzen, wobei die verwendeten Abkürzungen folgende Bedeutung haben:
- Fp. =: Schmelzpunkt
- X =: kristallin
- S =: smektisch
- S_{C} =: smektisch C
- S_{A} =: smektisch A
- N =: nematisch
- I =: isotrop

### Beispiel 1:

### 5-Brom-2-[4-(phenylmethoxy)phenyl]pyrimidin

104,25 g (0,438 Mol) 2,5-Dibrompyrimidin, 100,00 g (0,438 Mol) 4-(Phenylmethoxy)benzolboronsäure, 4,75 g (0,00438 Mol) Palladium (10 %) auf Aktivkohle, 4,50 g (0,01752 Mol) Triphenylphosphin und 93,00 g (0,876 Mol) Natriumcarbonat werden in 1000 ml Toluol, 500 ml Ethanol und 300 ml Wasser für 24 h auf 80°C erhitzt. Anschließend wird bei 80°C der Palladiumkatalysator durch Filtration von Reaktionsgemisch abgetrennt. Die wäßrige Unterphase des Reaktionsgemischs wird bei 80°C abgetrennt, bevor die organische Phase am Rotationsverdampfer von den Lösemitteln befreit und im Hochvakuum getrocknet wird. Das so erhaltene Rohprodukt wird aus Acetonitril (3000 ml) umkristallisiert, wonach 150,07 g (97 % Ausbeute, bezogen auf 2,5-Dibrompyrimidin) 5-Brom-2-[4-(phenylmethoxy)phenyl]pyrimidin (Gehalt nach HPLC: 98 %) erhalten werden. 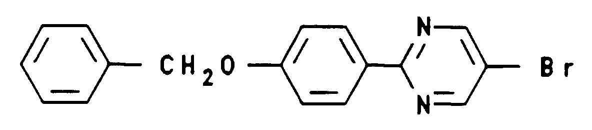 Fp.: 153-155°C

### Vergleichsbeispiel 1:

### Synthese von 5-Brom-2-[4-(phenylmethoxy)phenyl]-pyrimidin nach dem in DE 39 30 663 beschriebenen Verfahren

4,17 g (17,54 mmol) 2,5-Dibrompyrimidin, 4,00 g (17,54 mmol) 4-(Phenylmethoxy)benzolboronsäure, 0,19 g (0,175 mmol) Palladium (10 %) auf Aktivkohle und 3,72 g (35,10 mmol) Natriumcarbonat werden in 42 ml Toluol, 21 ml Ethanol und 12,5 ml Wasser analog Beispiel 1 umgesetzt.
Die Analyse (HPLC) des Rohprodukts zeigt ein Gemisch, welches neben den Edukten 2,5-Dibromyrimidin, 4-(Phenylmethoxy)benzolboronsäure und nicht identifizierten Produkten 6,3 % des angestrebten Produktes 5-Brom-2-[4-(phenylmethoxy)phenyl]pyrimidin enthält.

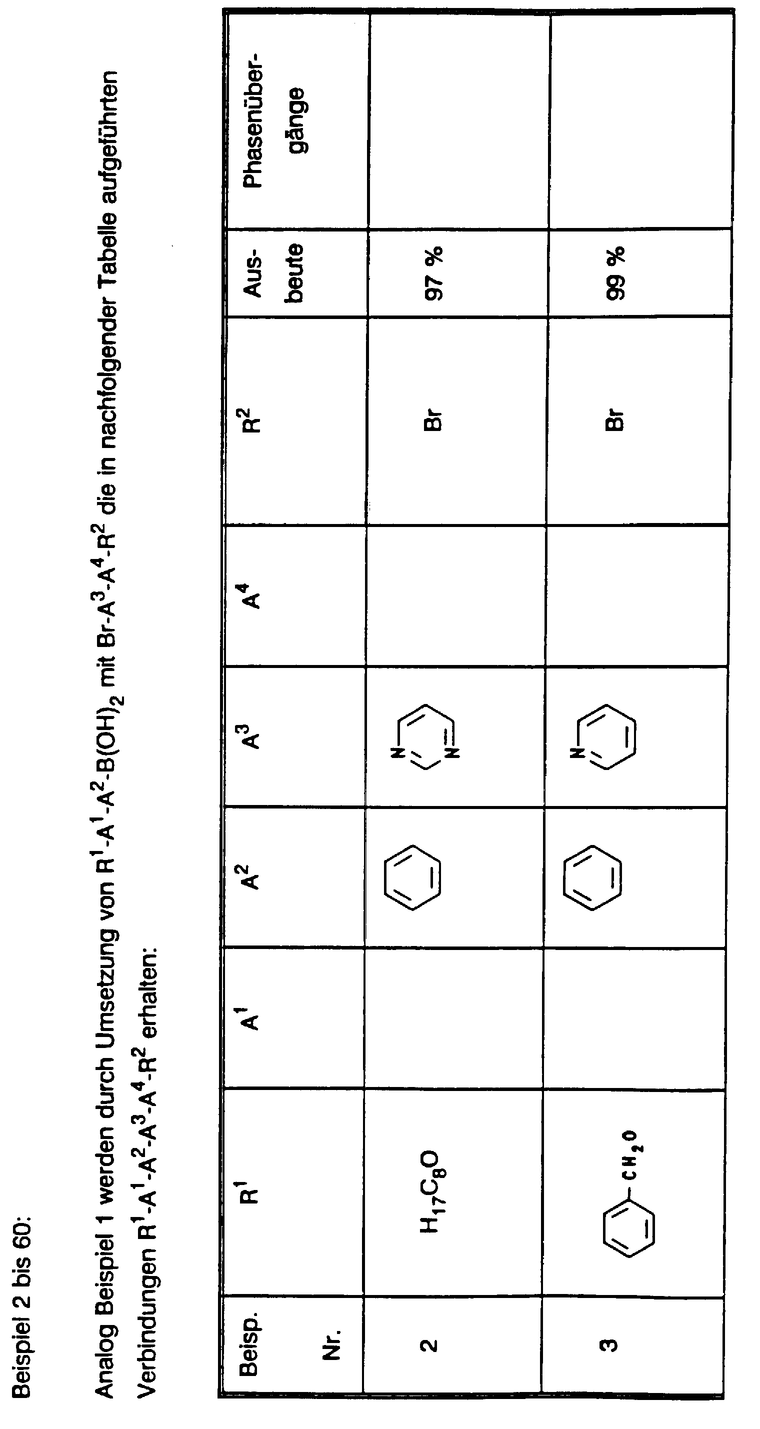

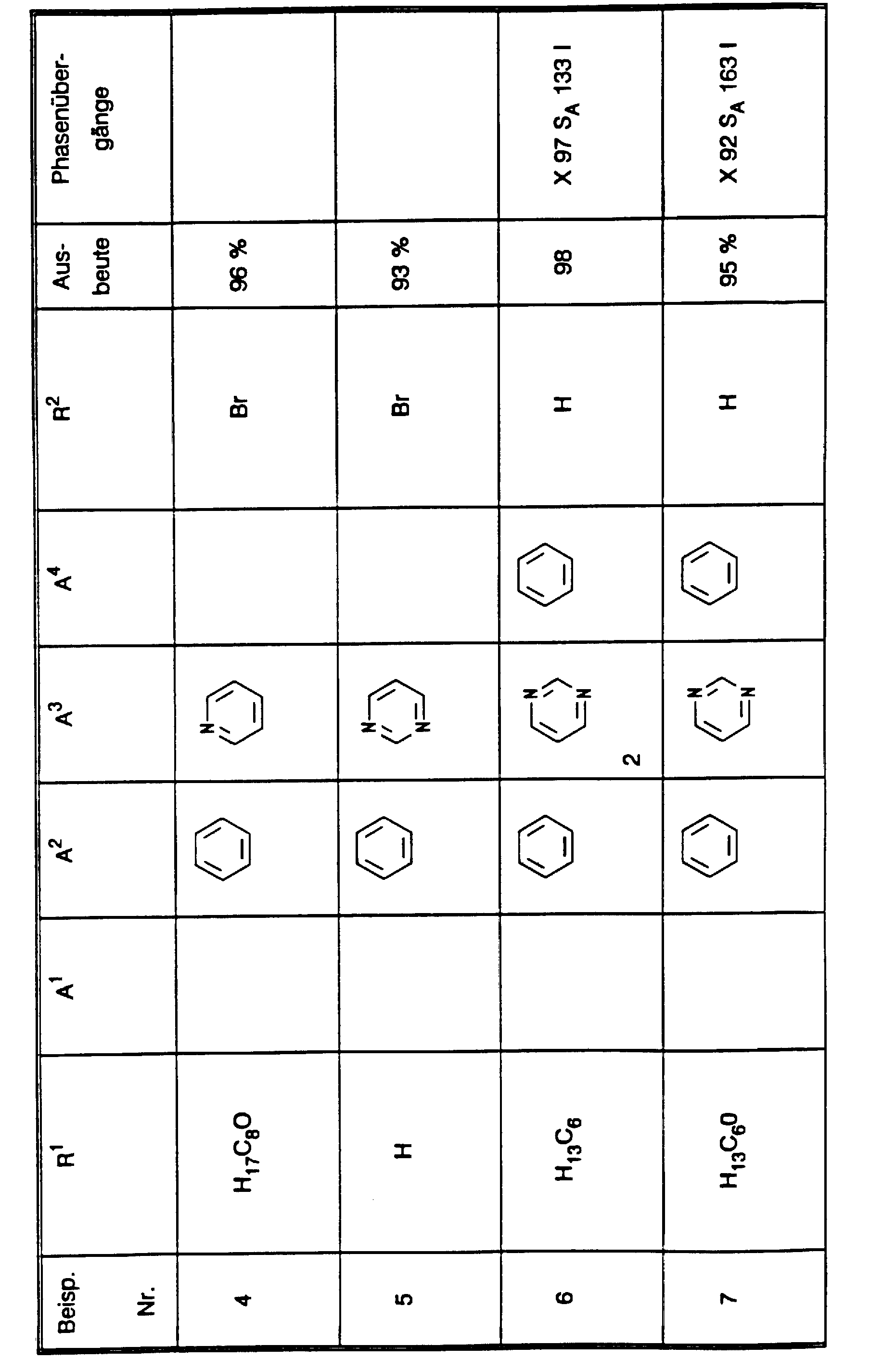

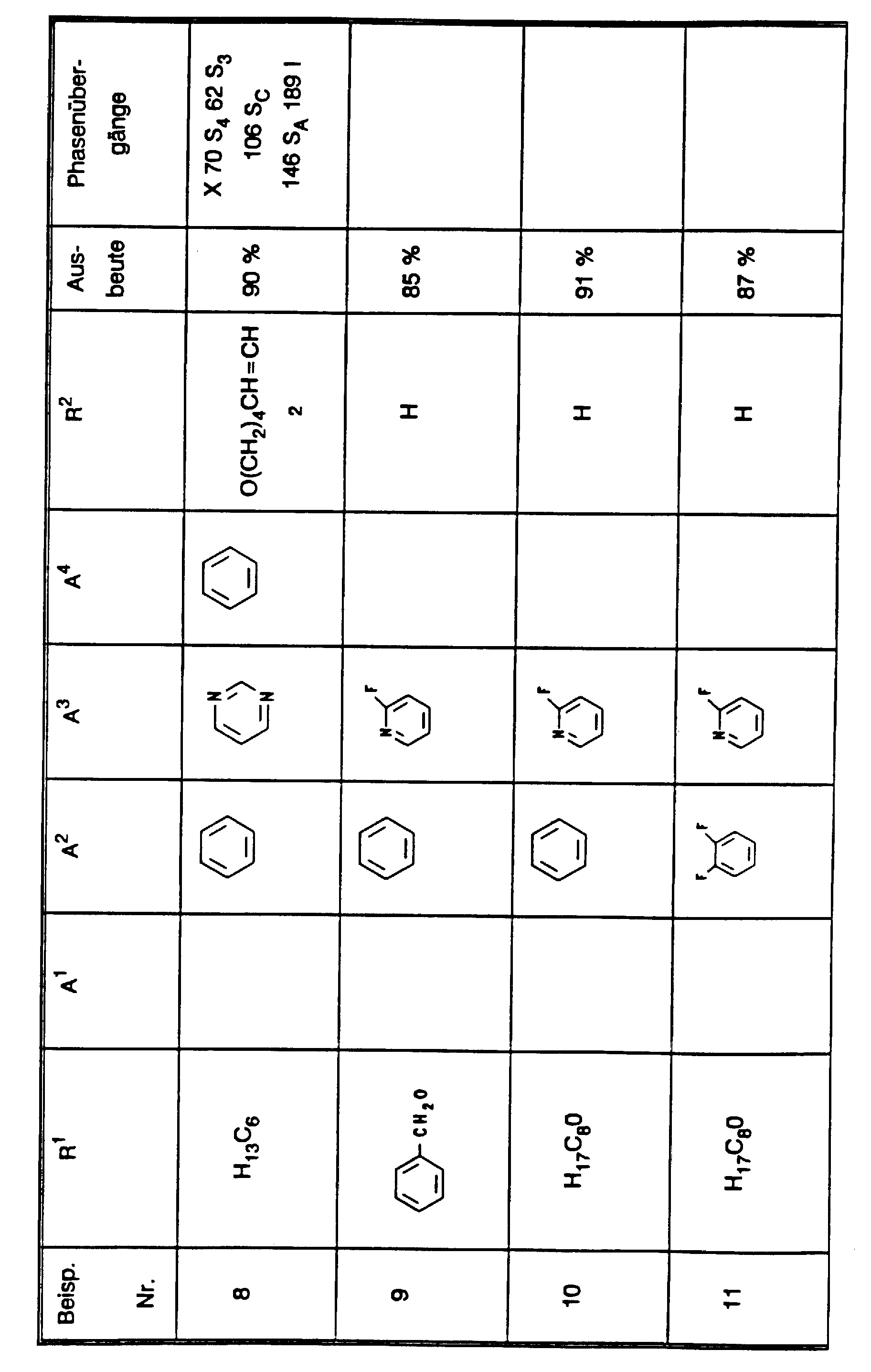

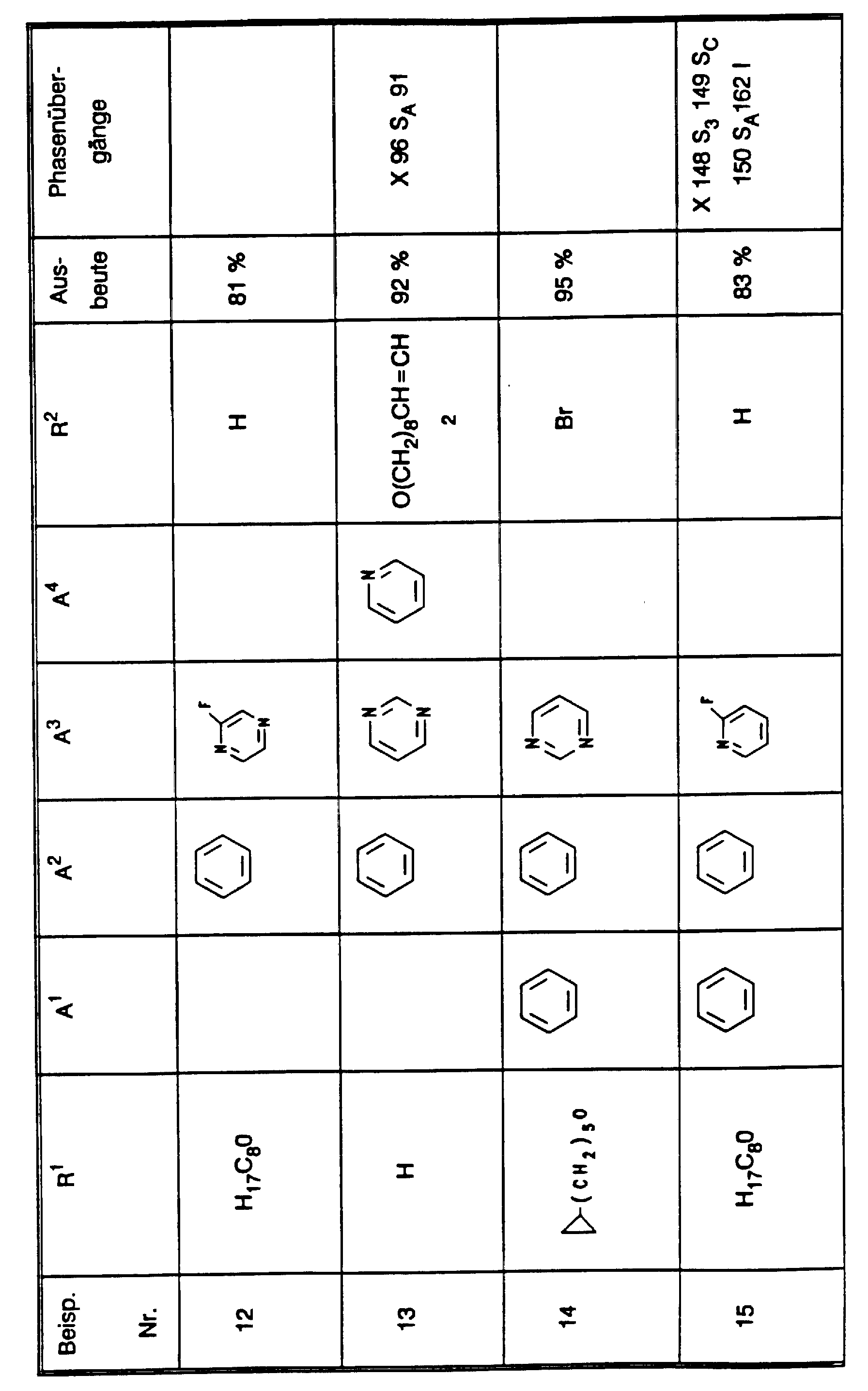

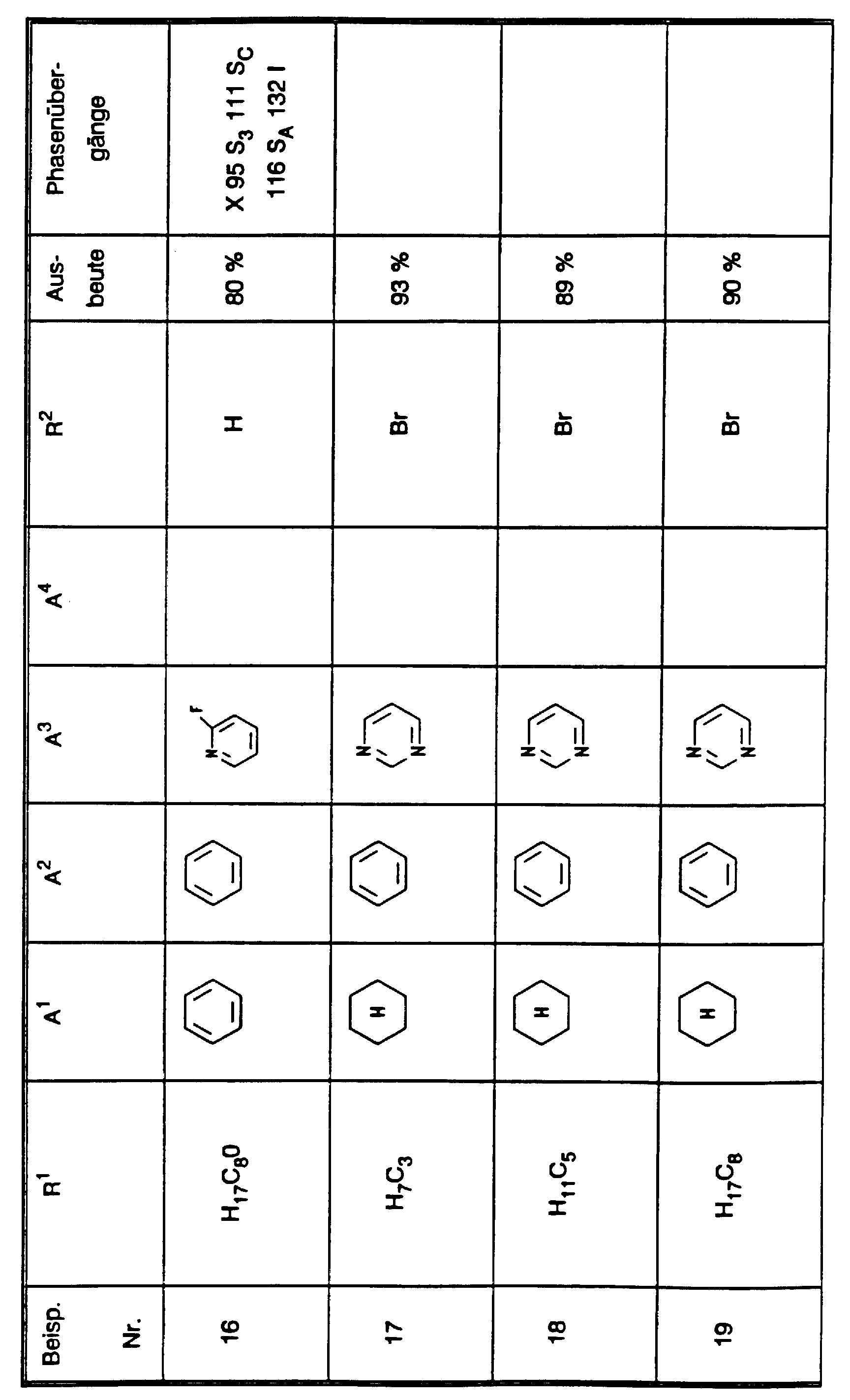

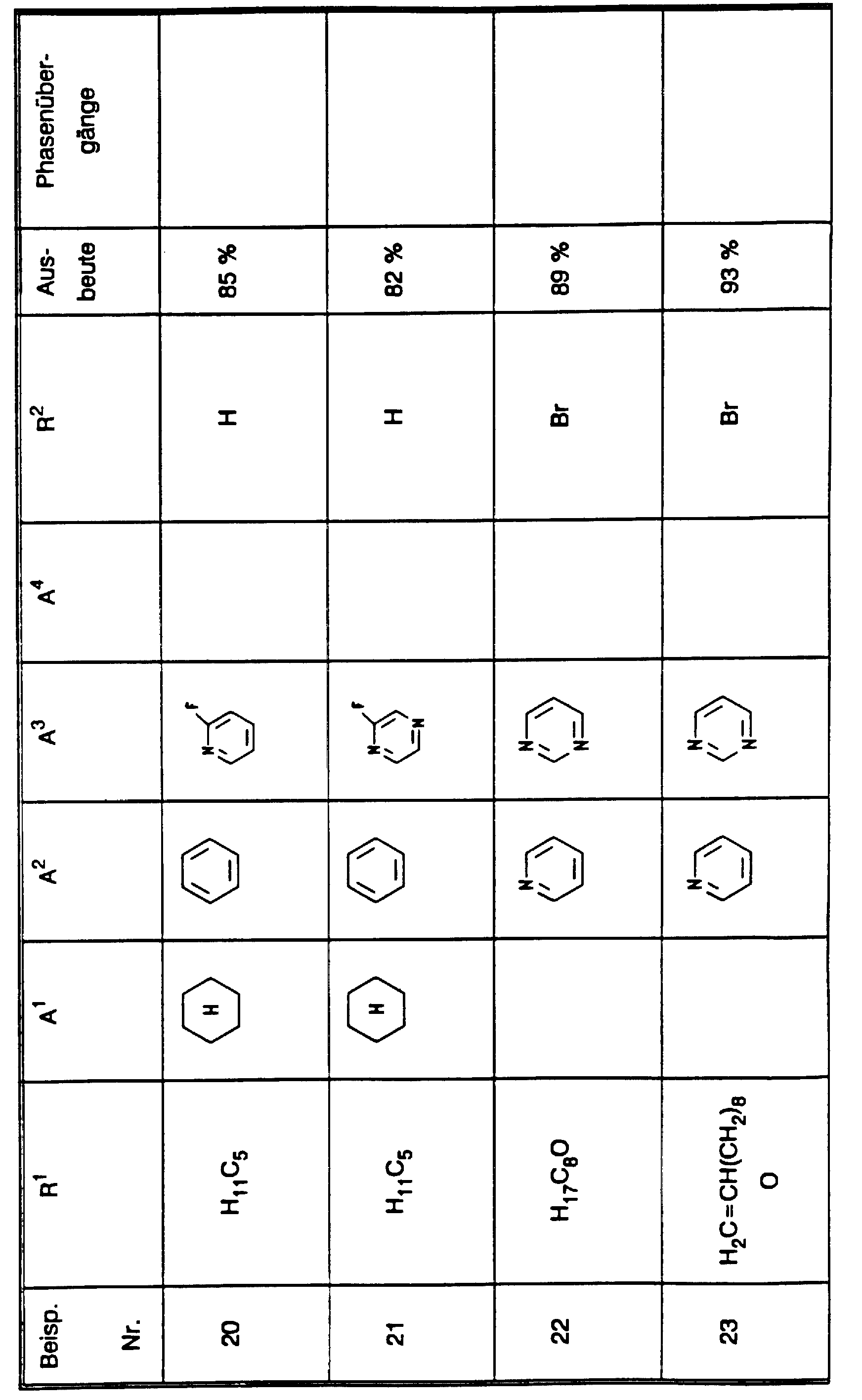

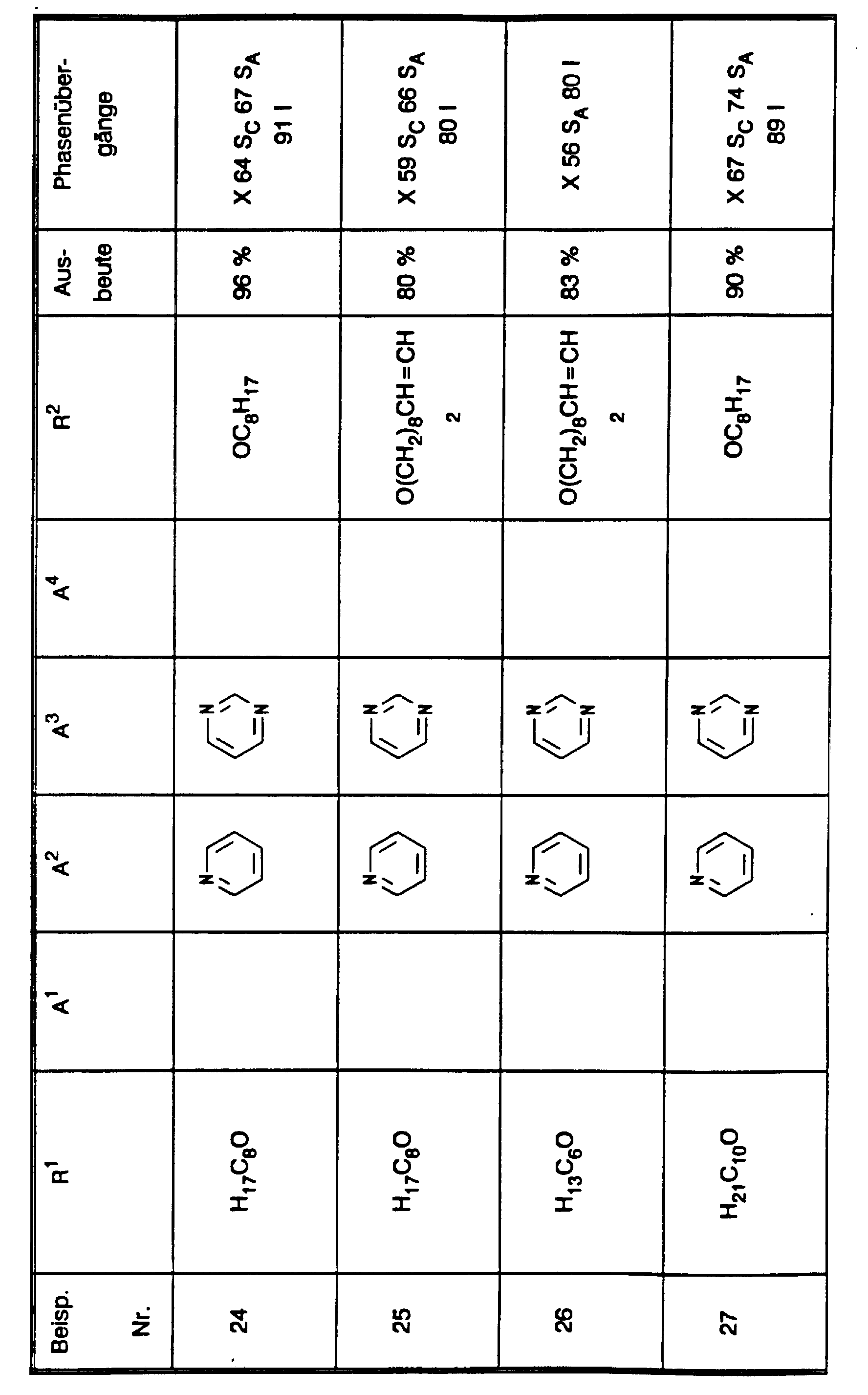

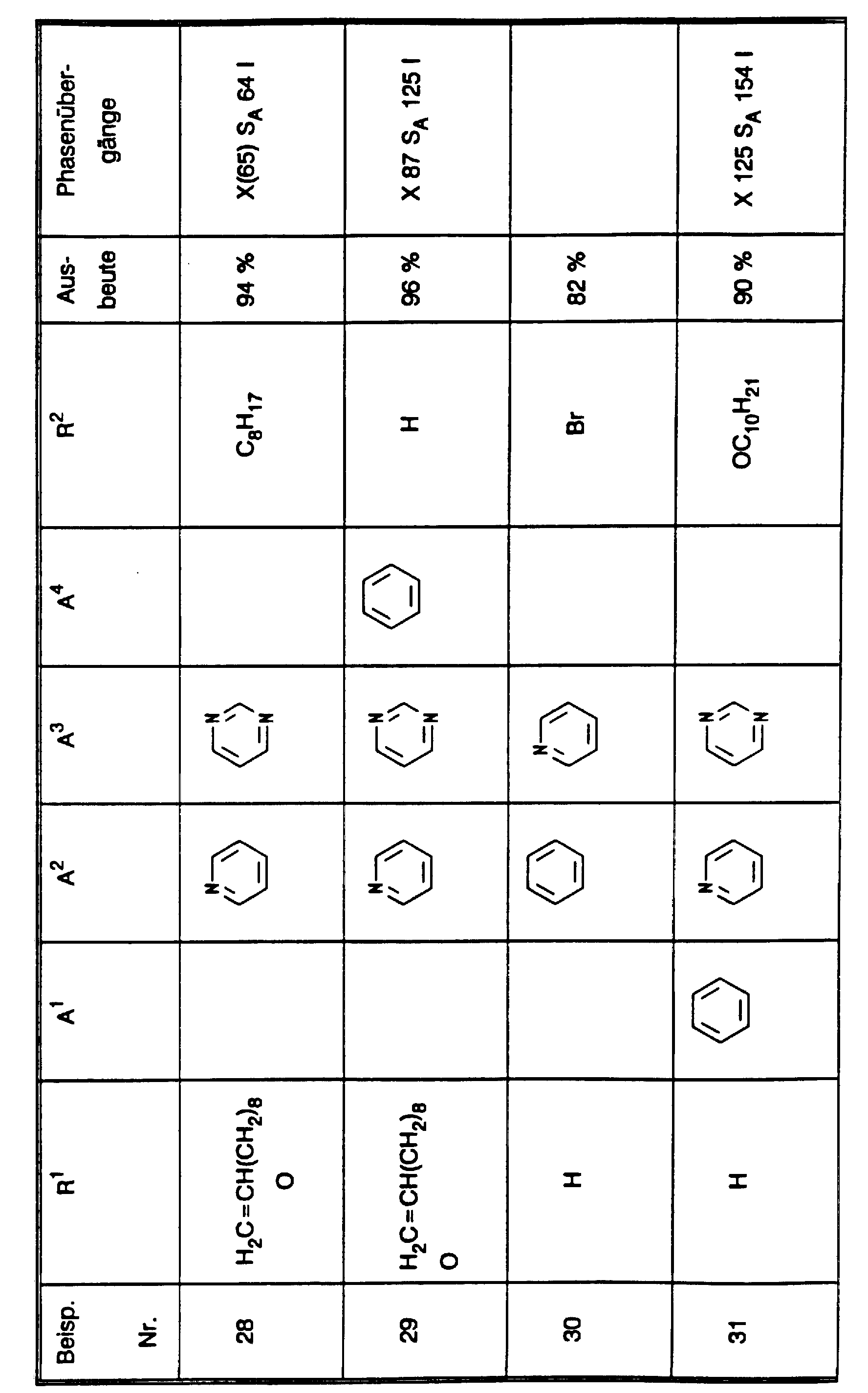

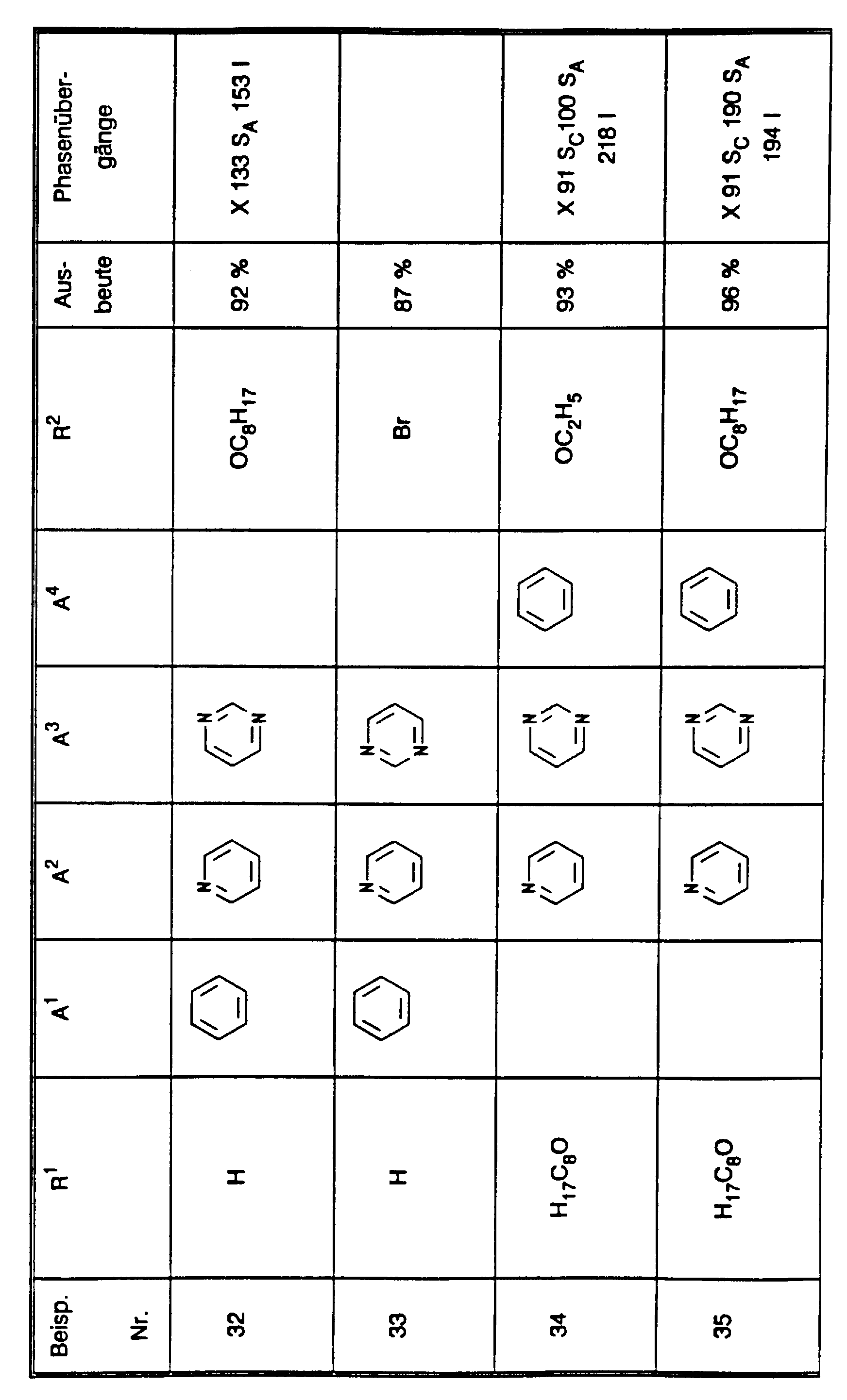

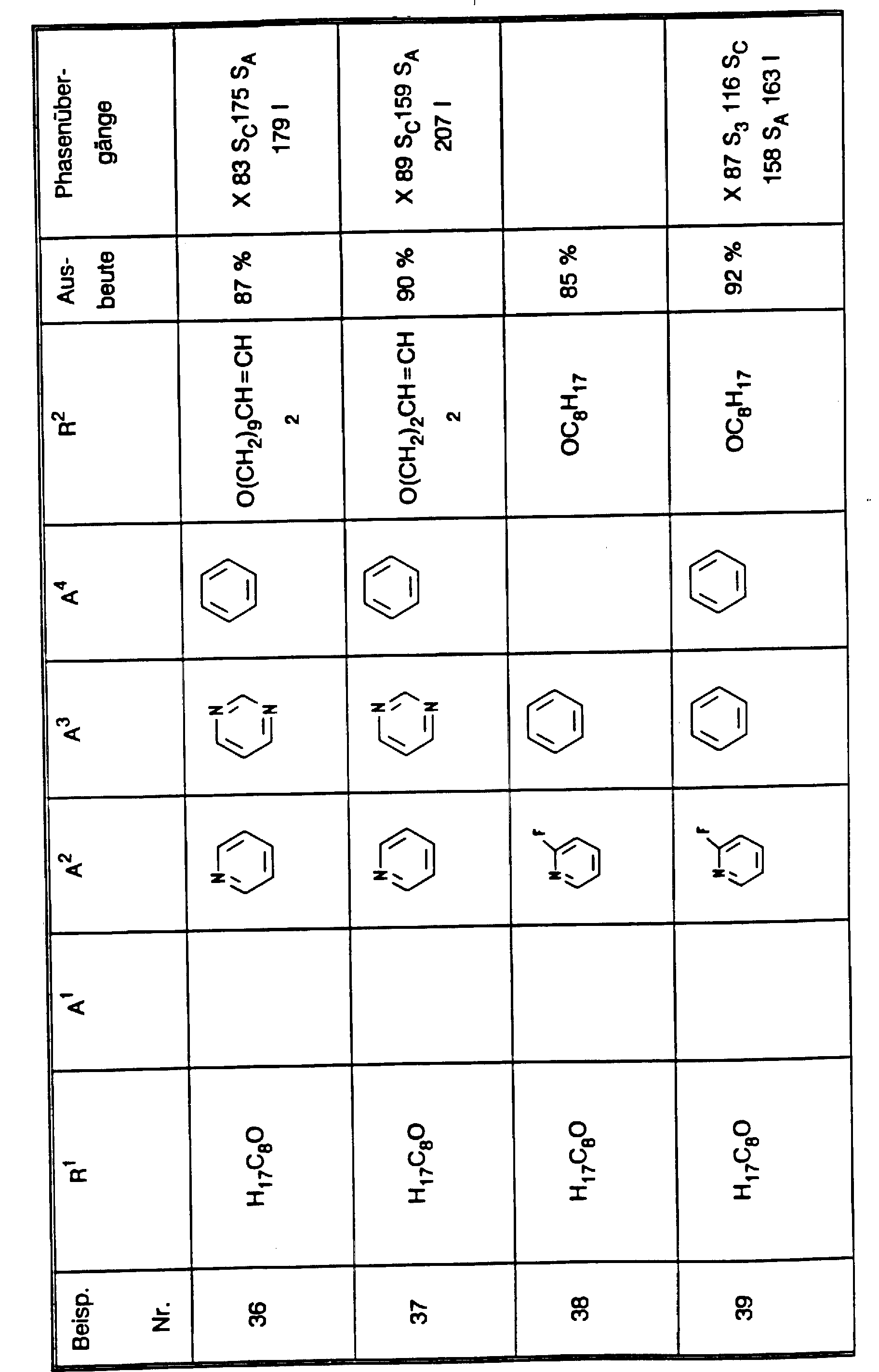

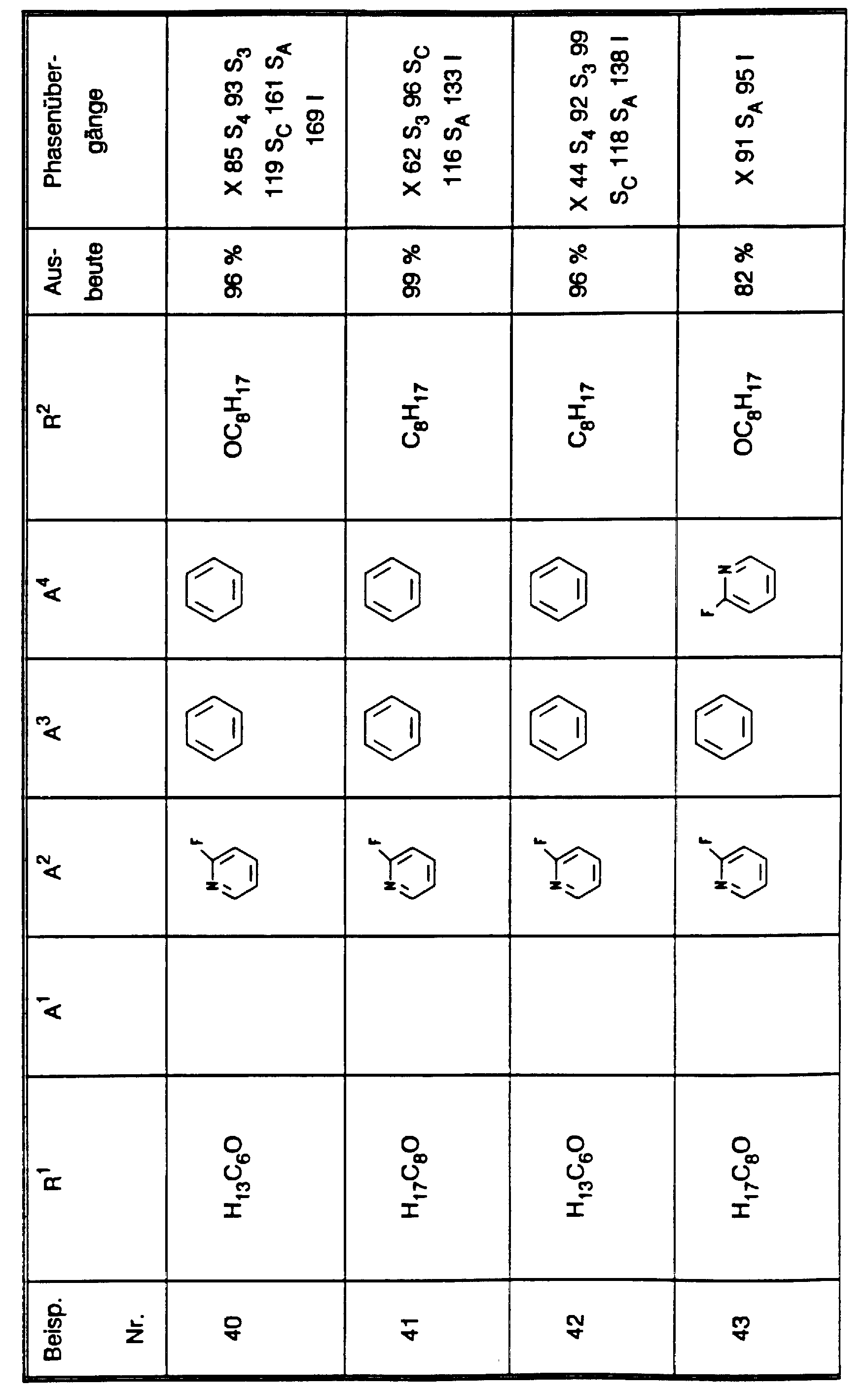

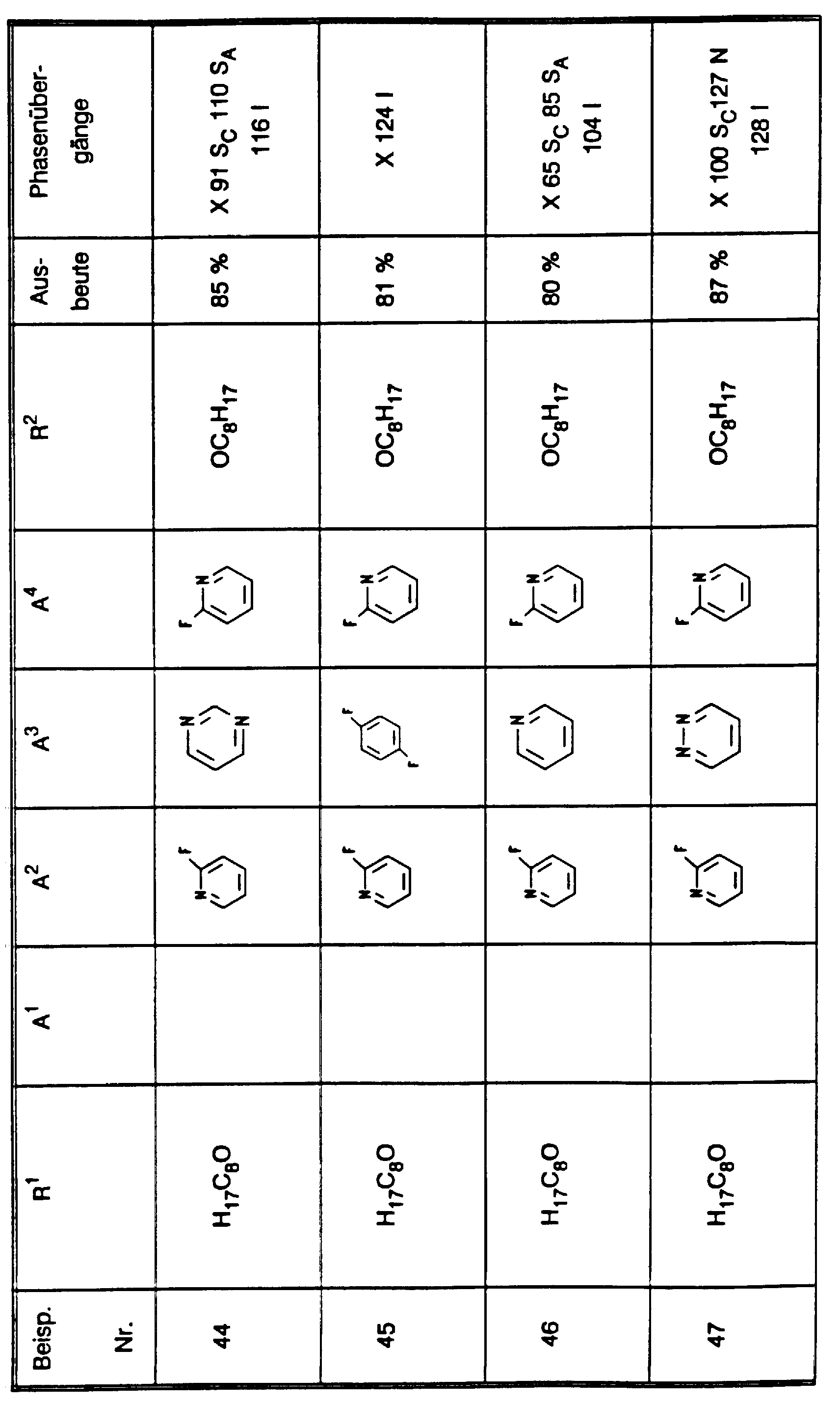

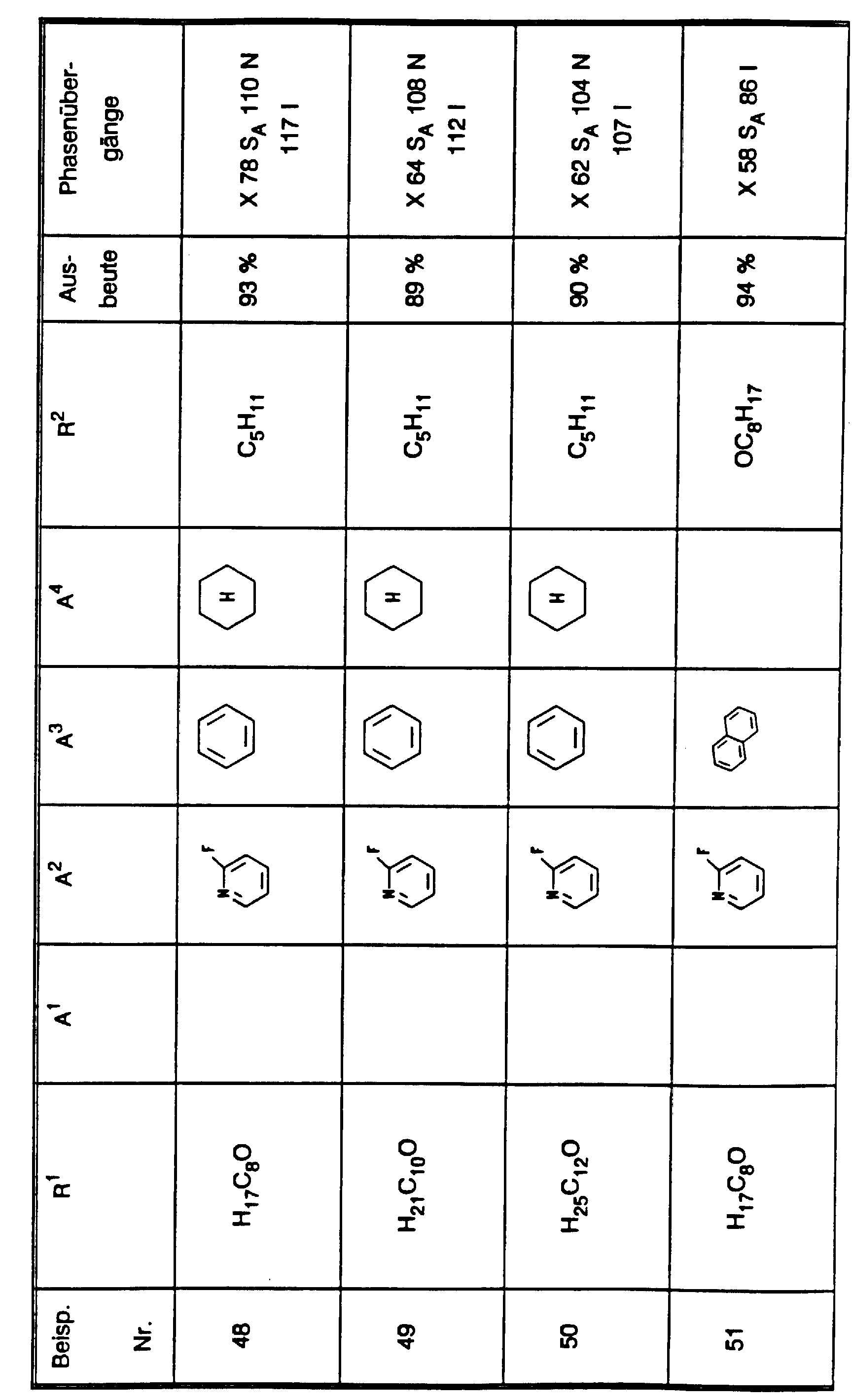

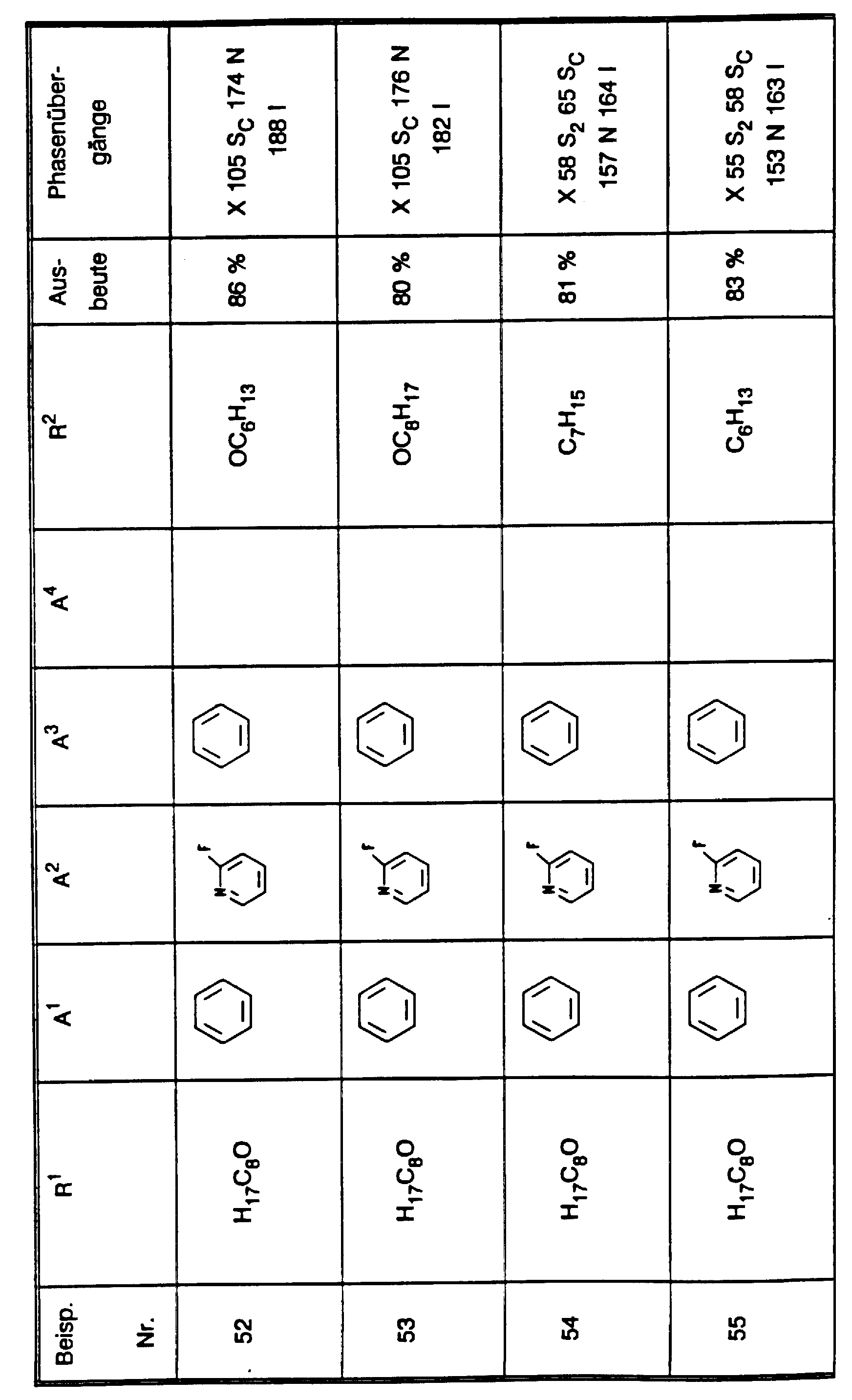

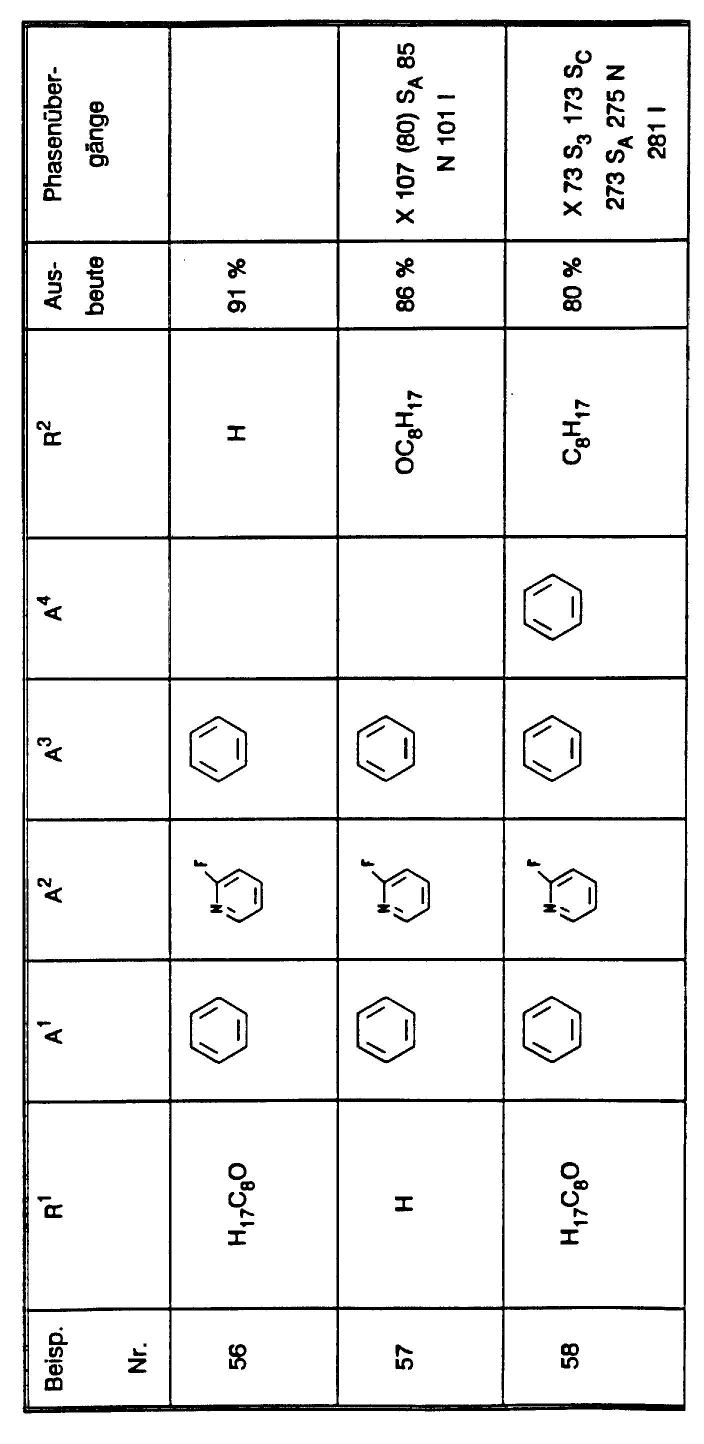

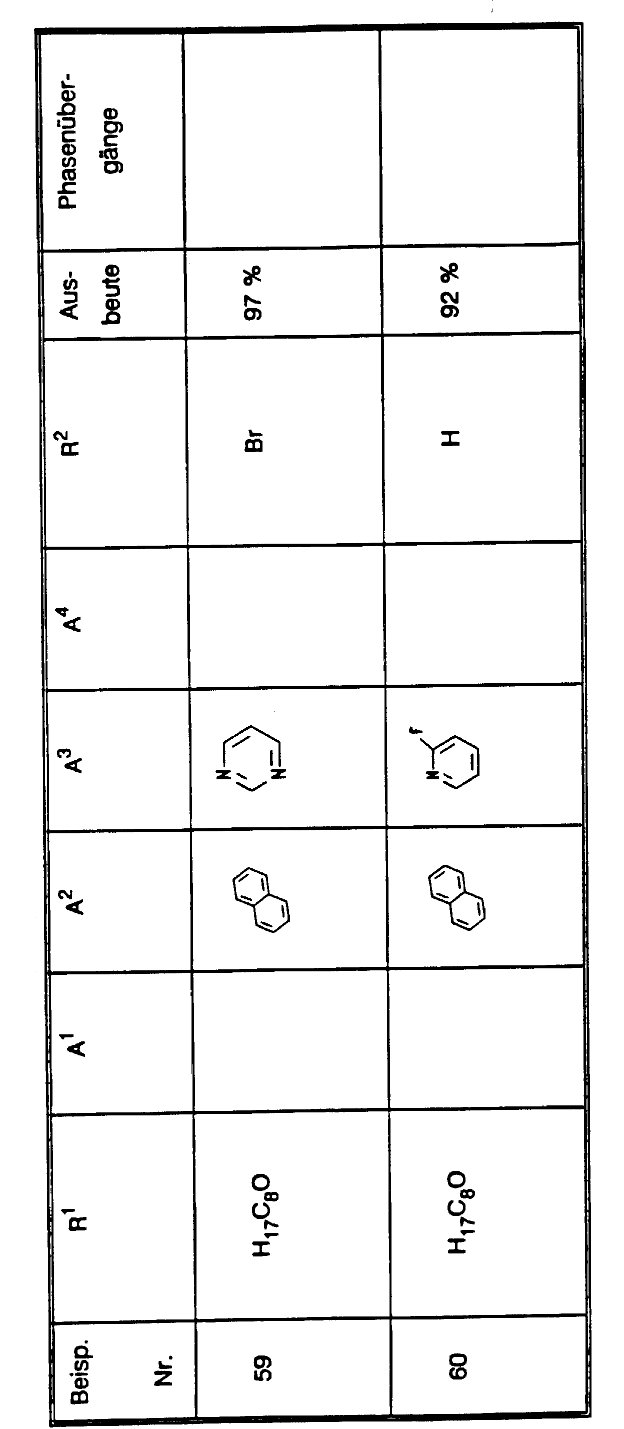

## Patentansprüche

1. Verfahren zur Herstellung von mehrkernigen aromatischen Verbindungen durch Kreuzkupplung von aromatischen Boronsäuren mit aromatischen Halogenverbindungen oder Perfluoralkylsulfonaten in Gegenwart von metallischem gegebenenfalls auf ein Trägermaterial aufgebrachtem Palladium als Katalysator, dadurch gekennzeichnet, daß die Kupplung in Gegenwart eines Liganden aus der Gruppe Phosphine, Diketone und tertiäre Amine und einer Base durchqeführt wird.

2. Verfahren nach Anspruch 1, dadurch gekennzeichnet, daß eine mehrkernige aromatische Verbindung der Formel I dargestellt wird
R¹(-A¹)ₖ(-M¹)ₗ-A²-A³(-M²)ₘ(-A⁴)ₙ-R² (I)
wobei R¹ und R² unabhängig voneinander Benzyloxy, H, F, Cl, Br, -NC, -CN,-CF₃, -OCF₃ oder ein geradkettiger oder verzweigter (mit oder ohne asymmetrisches C-Atom) Alkylrest mit 1 bis 18 C-Atomen sein können, wobei auch eine oder zwei nicht benachbarte -CH₂-Gruppen durch -O-, -S-, -CO-, -CO-O-, -O-CO-, -CO-S-, -S-CO-, -O-CO-O-, -CH=CH-, -C≡C-, 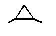 oder -Si(CH₃)₂- ersetzt sein können, und wobei auch ein oder mehrere H-Atome des Alkylrestes durch F, Cl, Br oder CN substituiert sein können, A¹ und A⁴ jeweils unabhängig voneinander 1,4-Phenylen, Pyrazin-2,5-diyl, Pyridazin-3,6-diyl, Pyridin-2,5-diyl, Pyrimidin-2,5-diyl, wobei eine oder zwei H-Atome durch Halogenatome, Cyano- und oder Methylgruppen substituiert sein können, trans-1,4-Cyclohexylen, worin eine oder zwei nicht benachbarte CH₂-Gruppen durch -O- oder -S- ersetzt sein können und worin ein oder zwei H-Atome durch Halogenatome, Cyano- und/oder Methylgruppen substituiert sein können, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Piperidin-1,4-diyl, Naphthalin-2,6-diyl, Bicyclo[2.2.2]octan-1,4-diyl, 1,3-Dioxaborinan-2,5-diyl oder trans-Dekalin-2,6-diyl sein können,
A² und A³ jeweils unabhängig voneinander 1,4-Phenylen, Pyrazin-2,5-diyl, Pyridazin-3,6-diyl, Pyridin-2,5-diyl, Pyrimidin-2,5-diyl, wobei ein oder zwei H-Atome durch Halogenatome, Cyano- und/oder Methylgruppen substituiert sein können, 1,3,4-Thiadiazol-2,5-diyl, 1,3-Thiazol-2,4-diyl, 1,3-Thiazol-2,5-diyl, Thiophen-2,4-diyl, Thiophen-2,5-diyl oder Naphthalin-2,6-diyl sein können,
M¹ und M² jeweils unabhängig voneinander
-O-, -S-, -CO-, -CO-O-, O-CO-, -CO-S, -S-CO-, -O-CO-O-, -CH₂-O, -OCH₂-, -CH₂CH₂-, -CH=CH-, -C≡C-, -CH(CN)-CH₂-, -CH₂-CH(CN)-, -CH=N-, -N=CH-,-CH₂CH₂CH₂-O-, -OCH₂CH₂CH₂-, -CH₂CH₂CO-O-, -OCOCH₂CH₂- sein können,
k, l, m, n jeweils unabhängig voneinander Null oder Eins bedeuten, indem man Boronsäuren der Formel II
R¹(-A¹)ₖ(-M¹)ₗ-A²-B(OH)₂ (II),
wobei R¹, A¹, A², M¹, k und l die oben angegebene Bedeutung haben, mit einer Verbindung der Formel (III)
X-A³(-M²)ₘ(-A⁴)ₙ-R² (III),
wobei R², A³, A⁴, M², m und n die oben angegebene Bedeutung haben, und X Chlor, Brom, Jod oder OSO₂-CₚF₂ₚ₊₁, worin p einen ganzzahligen Wert von 1 bis 10 darstellt, umsetzt.

3. Verfahren nach Anspruch 1 und/oder 2, dadurch gekennzeichnet, daß der Ligand in einem Anteil von 0,1-20 Mol-%, bezogen auf die aromatische Halogenverbindung oder das aromatische Perfluoralkylsulfonat, zugesetzt wird.

4. Verfahren nach einem oder mehreren der vorhergehenden Anspruche, dadurch gekennzeichnet, daß als Base mindestens eine Verbindung aus der Gruppe Alkali und Erdalkalimetallhydroxide, Alkali- und Erdalkalimetallcarbonate, Alkalimetallhydrogencarbonate, Alkali- und Erdalkalimetallacetate, Alkali- und Erdalkalimetallalkloholate und primäre, sekundäre und tertiäre Amine verwendet wird.

5. Verfahren nach Anspruch 4, dadurch gekennzeichnet, daß die Base in einem Anteil von 100-500 Mol.-%, bezogen auf die aromatische Boronsäure, verwendet wird.

6. Verfahren nach einem oder mehreren der vorhergehenden Ansprüche, dadurch gekennzeichnet, daß es bei Temperaturen zwischen 50 und 150°C durchgeführt wird.

7. Verfahren nach einem oder mehreren der vorhergehenden Ansprüche, dadurch gekennzeichnet, daß die Kreuzkupplungsreaktion in einem Lösungsmittel oder Lösungsmittelgemisch durchgeführt wird, das mindestens eine Verbindung aus der Gruppe Ether, Kohlenwasserstoffe, Alkohole, Ketone, Amide, Nitrile und Wasser enthält.

8. Verfahren nach einem oder mehreren der vorhergehenden Ansprüche, dadurch gekennzeichnet, daß als Katalysator Palladium in pulverisierter Form, Palladium auf Aktivkohle, Palladium auf Aluminiumoxid, Palladium auf Bariumsulfat oder Palladium auf Calciumcarbonat, jeweils mit einem Palladiumgehalt von 0,5 bis 10 Gew.-%, verwendet wird.

## Claims

1. A process for preparing polycyclic aromatic compounds by cross-coupling aromatic boronic acids with aromatic halogen compounds or perfluoroalkylsulfonates in the presence of metallic palladium, if desired applied to a support material, as catalyst, wherein the coupling is carried out in the presence of a ligand selected from the group consisting of phosphines, diketones and tertiary amines and a base.

2. The process as claimed in claim 1, wherein a polycyclic aromatic compound of the formula I
R¹(-A¹)ₖ(-M¹)ₗ-A²-A³(-M²)ₘ(-A⁴)ₙ-R² (I)
where R¹ and R² can be, independently of one another, benzyloxy, H, F, Cl, Br, -NC, -CN, -CF₃, -OCF₃ or a straight-chain or branched alkyl radical (with or without an asymmetric carbon atom) having from 1 to 18 carbon atoms, where one or two nonadjacent -CH₂ groups can also be replaced by -O-, -S-, -CO-, -CO-O-, -O-CO-, -CO-S-, -S-CO-, -O-CO-O-, -CH=CH-, -C≡C-, 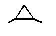 or -Si(CH₃)₂-, and where one or more hydrogen atoms of the alkyl radical can also be replaced by F, Cl, Br or CN, A¹ and A⁴ can each be, independently of one another, 1,4-phenylene, pyrazine-2,5-diyl, pyridazine-3,6-diyl, pyridine-2,5-diyl, pyrimidine-2,5-diyl, where one or two hydrogen atoms can be replaced by halogen atoms, cyano and/or methyl groups, trans-1,4-cyclohexylene where one or two nonadjacent CH₂ groups can be replaced by -O- or -S- and where one or two hydrogen atoms can be replaced by halogen atoms, cyano and/or methyl groups, (1,3,4)-thiadiazole-2,5-diyl, 1,3-thiazole-2,4-diyl, 1,3-thiazole-2,5-diyl, thiophene-2,4-diyl, thiophene-2,5-diyl, piperazine-1,4-diyl, piperazine-2, piperidine-1,4-diyl, naphthalene-2,6-diyl, bicyclo[2.2.2]octane-1,4-diyl, 1,3-dioxaborinane-2,5-diyl or trans-decalin-2,6-diyl,
A² and A³ can each be, independently of one another, 1,4-phenylene, pyrazine-2,5-diyl, pyridazine-3,6-diyl, pyridine-2,5-diyl, pyrimidine-2,5-diyl, where one or two hydrogen atoms can be replaced by halogen atoms, cyano and/or methyl groups, 1,3,4-thiadiazole-2,5-diyl, 1,3-thiazole-2,4-diyl, 1,3-thiazole-2,5-diyl, thiophene-2,4-diyl, thiophene-2,5-diyl or naphthalene-2,6-diyl,
M¹ and M² can each be, independently of one another, -O-, -S-, -CO-, -CO-O-, -O-CO-, -CO-S-, -S-CO-, -O-CO-O-, -CH₂-O-, -OCH₂-, -CH₂CH₂-, -CH=CH-, -C≡C-, -CH(CN)-CH₂- -CH₂-CH(CN)-, -CH=N-, -N=CH-, -CH₂CH₂CH₂-O- , -OCH₂CH₂CH₂-, CH₂CH₂CO-O- , -O-COCH₂CH₂-,
k, l, m, n are each, independently of one another, zero or one,
is prepared by reacting boronic acids of the formula II
R¹(-A¹)ₖ(-M¹)ₗ-A²-B(OH)₂ (II)
where R¹, A¹, A², M¹, k and l are as defined above, with a compound of the formula (III)
X-A³(-M²)ₘ(-A⁴)ₙ-R² (III)
where R², A³, A⁴, M², m and n are as defined above, and X is chlorine, bromine, iodine or OSO₂-CₚF₂ₚ₊₁, where p is an integer from 1 to 10.

3. The process as claimed in claim 1 and/or 2, wherein the ligand is added in a proportion of 0.1-20 mol %, based on the aromatic halogen compound or the aromatic perfluoroalkylsulfonate.

4. The process as claimed in one or more of the preceding claims, wherein the base used is at least one compound selected from the group consisting of alkali metal and alkaline earth metal hydroxides, alkali metal and alkaline earth metal carbonates, alkali metal hydrogen carbonates, alkali metal and alkaline earth metal acetates, alkali metal and alkaline earth metal alkoxides and primary, secondary and tertiary amines.

5. The process as claimed in claim 4, wherein the base is used in a proportion of 100-500 mol %, based on the aromatic boronic acid.

6. The process as claimed in one or more of the preceding claims, carried out at temperatures between 50 and 150°C.

7. The process as claimed in one or more of the preceding claims, wherein the cross-coupling reaction is carried out in a solvent or solvent mixture which contains at least one compound selected from the group consisting of ethers, hydrocarbons, alcohols, ketones, amides, nitriles and water.

8. The process as claimed in one or more of the preceding claims, wherein the catalyst used is palladium in powdered form, palladium on activated carbon, palladium on aluminum oxide, palladium on barium sulfate or palladium on calcium carbonate, in each case having a palladium content of from 0.5 to 10% by weight.

## Revendications

1. Procédé pour la préparation de composés aromatiques polycycliques par copulation croisée des acides boroniques aromatiques avec des composés halogénés aromatiques ou des perfluoroalkylsulfonates en présence de palladium métal, éventuellement déposé sur une matière support, en tant que catalyseur, caractérisé en ce que l'on met en oeuvre la copulation en présence d'un ligand du groupe des phosphines, des dicétones et des amines tertiaires et d'une base.

2. Procédé selon la revendication 1, caractérisé en ce que l'on fait réagir un composé aromatique polycyclique de formule I représenté par la formule
R¹(-A¹)ₖ(-M¹)ₗ-A²-A³(-M²)ₘ(-A⁴)ₙ-R² (I)
R¹ et R², indépendamment l'un de l'autre, étant benzyloxy, H, F, CI, Br, -NC, -CN, -CF₃, -OCF₃ ou un radical alkyle linéaire ou ramifié (avec ou sans atomes de carbone asymétriques) avec 1 à 18 atomes de carbone, un ou deux groupes -CH₂ non voisins pouvant également être remplacés par -O-, -S-, -CO-, -CO-O-, -O-CO-,-CO-S-,-S-CO-,-O-CO-O-,-CH=CH-,-C≡C-, 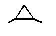 ou -Si(CH₃)₂-, et un ou plusieurs atomes de H du radical alkyle pouvant également être substitués par F, Cl, Br ou CN, A¹ et A⁴ représentent, indépendamment l'un de l'autre, 1,4-phénylène, pyrazine-2,5-diyle, pyridazine-3,6-diyle, pyridine-2,5-diyle, pyrimidine-2,5-diyle, un ou deux atomes de H pouvant être substitués par des atomes d'halogènes, des groupes cyano et/ou méthyle, représente aussi trans-1,4-cyclohexylène, où un ou deux groupes -CH₂ non voisins pouvant être remplacés par -O- ou -S- et où un ou deux atomes de H pouvant être substitués par des atomes d'halogènes, des groupes cyano et/ou méthyle, représentent aussi (1,3,4)-thiadiazol-2,5-diyle, 1,3-thiazol-2,4-diyle, 1,3-thiazol-2,5-diyle, thiophène-2,4-diyle, thiophène-2,5-diyle, pipérazine-1,4-diyle, pipérazine-2,5-diyle, pipéridine-1,4-diyle, naphtalène-2, 6-diyle, bicyclo-[2.2.2]octane-1,4-diyle, 1,3-dioxaborinane-2,5-diyle ou trans-décaline-2,6-diyle, A² et A³ représentent chacun, indépendamment l'un de l'autre, 1,4-phénylène, pyrazine-2,5-diyle, pyridazine-3,6-diyle, pyridine-2,5-diyle, pyrimidine-2,5-diyle, un ou deux atomes de H pouvant être substitués par des atomes d'halogènes, des groupes cyano et/ou méthyle, représetent 1,3,4-thiadiazol-2,5-diyle, 1,3-thiazol-2,4-diyle, 1,3-thiazol-2,5-diyle, thiophène-2,4-diyle, thiophène-2,5-diyle ou naphtalène-2,6-diyle.
M¹ et M² représentent chacun, indépendamment l'un de l'autre, -O-, -S-, -CO-, -CO-O-, O-CO-, -CO-S-, -S-CO-, -O-CO-O-, -CH₂O-, -OCH₂, -CH₂-CH₂-, -CH=CH-, -C≡C-, -CH(CN)-CH₂-, -CH₂-CH(CN)-, -CH=N-, -N=CH-, CH₂CH₂CH₂-O-, -O-CH₂CH₂CH₂-, -CH₂CH₂CO-O-, -O-COCH₂CH₂-, et
k, l, m, n représentent chacun, indépendamment l'un de l'autre, 0 ou 1, l'acide boronique de formule II
R¹(-A¹)ₖ(-M¹)ₗ-A²-B(OH)₂ (II)
R¹, A¹, A², M¹, k et l ayant les significations données ci-dessus avec un composé de formule III
X-A³(-M²)ₘ(-A⁴)ₙ-R₂ (III)
R², A³, A⁴, M², m et n ayant les significations données ci-dessus et X représente le chlore, le brome, l'iode ou OSO₂-CₚF₂ₚ₊₁, où p est un nombre entier de 1 à 10.

3. Procédé selon la revendication 1 et/ou 2, caractérisé en ce que l'on ajoute le ligand à un taux de 0,1 à 20 % en moles par rapport aux composés halogénés aromatiques ou le perfluoroalkylsulfonate aromatique.

4. Procédé selon une ou plusieurs des revendications précédentes, caractérisé en ce que l'on utilise en tant que base au moins un composé du groupe des hydroxydes alcalins et de métaux alcalino-terreux, de carbonates alcalins et de métaux alcalino-terreux, de bicarbonates alcalins, des acétates de métaux alcalins et alcalino-terreux, des alcoolates de métaux alcalins et alcalino-terreux et des amines primaires, secondaires et tertiaires.

5. Procédé selon la revendication 4, caractérisé en ce que l'on utilise la base à un taux de 100 à 500 % en moles par rapport à l'acide boronique aromatique.

6. Procédé selon une ou plusieurs des revendications précédentes, caractérisé en ce qu'on le met en oeuvre à des températures comprises entre 50 et 150 °C.

7. Procédé selon une ou plusieurs des revendications précédentes, caractérisé en ce que l'on met en oeuvre la réaction de copulation croisée dans un solvant ou un mélange de solvants, qui contient au moins un composé pris dans le groupe comportant les éthers, les hydrocarbures chlorés, les alcools, les cétones, les amides, les nitriles et l'eau.

8. Procédé selon une ou plusieurs des revendications précédentes, caractérisé en ce que l'on utilise en tant que catalyseur le palladium sous forme pulvérisée, le palladium sur du charbon actif, le palladium sur de l'oxyde d'aluminium, le palladium sur du sulfate de baryum ou le palladium sur du carbonate de calcium, toujours avec une teneur en palladium de 0,5 à 10 % en poids.
